(19) Europäisches Patentamt
European Patent Office
Office européen des brevets

(11) EP 2 872 107 B1

(12) EUROPEAN PATENT SPECIFICATION

(45) Date of publication and mention of the grant of the patent:
**21.07.2021 Bulletin 2021/29**

(21) Application number: **13728215.8**

(22) Date of filing: **13.06.2013**

(51) Int Cl.:
*A61K 8/25* (2006.01) *A61K 8/29* (2006.01)
*A61Q 17/04* (2006.01) *A61K 8/49* (2006.01)
*A61K 8/81* (2006.01) *A61K 8/02* (2006.01)

(86) International application number:
**PCT/EP2013/062247**

(87) International publication number:
**WO 2014/009097 (16.01.2014 Gazette 2014/03)**

(54) **COSMETIC COMPOSITION CONTAINING SCREENING COMPOSITE PARTICLES**

KOSMETISCHE ZUSAMMENSETZUNG ENTHALTEND FILTRIERENDE VERBUNDTEILCHEN

COMPOSITION COSMETIQUE COMPRENANT DES PARTICULES COMPOSITES FILTRANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2012 FR 1256759**
**22.10.2012 US 201261716922 P**

(43) Date of publication of application:
**20.05.2015 Bulletin 2015/21**

(73) Proprietor: **L'Oréal**
**75008 Paris (FR)**

(72) Inventors:
- **WILLIEN, Maud**
  **F-94400 Vitry Sur Seine (FR)**
- **ROUDOT, Angelina**
  **F-94270 Le Kremlin Bicêtre (FR)**
- **CANDAU, Didier**
  **F-91570 Bievres (FR)**

(74) Representative: **L'Oreal**
**Service D.I.P.I.**
**9, rue Pierre Dreyfus**
**92110 Clichy (FR)**

(56) References cited:
**EP-A1- 2 229 931**

- **DATABASE WPI Week 200801 Thomson Scientific, London, GB; AN 2008-A06839 XP002696942, & JP 2007 302647 A (DAITO KASEI KOGYO KK) 22 November 2007 (2007-11-22)**
- **DATABASE WPI Week 201248 Thomson Scientific, London, GB; AN 2012-A03495 XP002696943, & KR 2011 0137157 A (KOLMAR CO LTD) 22 December 2011 (2011-12-22)**
- **DATABASE WPI Week 201113 Thomson Scientific, London, GB; AN 2011-A83589 XP002696944, & KR 2011 0001539 A (KOLMAR CO LTD) 6 January 2011 (2011-01-06)**
- **"Eospoly", INTERNET CITATION, 2008, pages 1-2, XP002659381, Retrieved from the Internet: URL:http://www.creationscouleurs.com/as/as_eospoly_web.pdf [retrieved on 2011-09-05]**
- **"Stradivarious foundation (colour)", INTERNET CITATION, February 2010 (2010-02), pages 1-2, XP002659379, Retrieved from the Internet: URL:http://www.creationscouleurs.com/formulations/sun/spf_foundations/1406_2_2010StradivariusFoundationColour.pdf [retrieved on 2011-09-05]**
- **DATABASE WPI Week 200428 Thomson Scientific, London, GB; AN 2004-298340 XP002696945, & JP 2004 067624 A (KAO CORP) 4 March 2004 (2004-03-04)**
- **None**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The subject of the present invention is a composition containing, in a cosmetically acceptable medium, at least:

a) at least one aqueous phase and
b) composite particles A in spherical form, having a mean size of between 0.1 and 30 μm comprising at least one particulate UV-screening agent and a core constituted of at least one inorganic material and/or of at least one organic material, and
c) free particles B of inorganic UV-screening agent having a mean elementary size greater than 0.07 μm.

This composition is for topical use and is more particularly intended for the photoprotection of the skin and/or hair against ultraviolet (UV) radiation.

It is known that light radiation with wavelengths of between 280 nm and 400 nm permits tanning of the human epidermis and that light rays with wavelengths more particularly between 280 and 320 nm, known as UV-B rays, cause skin burns and erythema which can harm the development of a natural tan.

For these reasons, and also for aesthetic reasons, there is constant demand for means for controlling this natural tanning in order thus to control the colour of the skin; this UV-B radiation should thus be screened out.

It is also known that UV-A rays, with wavelengths between 320 and 400 nm, which cause tanning of the skin, are liable to induce adverse changes therein, in particular in the case of sensitive skin or skin that is continually exposed to solar radiation. UV-A rays cause in particular a loss of elasticity of the skin and the appearance of wrinkles leading to premature ageing of the skin.

Thus, for aesthetic and cosmetic reasons, for instance conservation of the skin's natural elasticity, people increasingly wish to control the effect of UV-A rays on their skin. It is thus desirable also to screen out UV-A radiation.

For the purpose of protecting the skin and keratin materials against UV radiation, antisun compositions comprising organic screening agents that are active in the UV-A range and in the UV-B range are generally used.

There are many cosmetic products comprising one or more inorganic and/or organic UV-screening agents. Fine inorganic particles based on a metal oxide such as titanium dioxide ($TiO_2$) are usually used for children's skin or sensitive skin, to protect the skin against UV rays.

These fine metal oxide particles generally have a mean elementary particle size of less than or equal to 0.1 μm, preferably between 0.005 and 0.1 μm, preferably between 0.01 and 0.1 μm, and preferentially between 0.015 and 0.05 μm.

Inorganic screening agents, such as metal oxide particles, and in particular titanium oxide particles, the primary particle size of which is greater than 0.07 μm on average, are used because of the high levels of protection that can be obtained by combining organic UV-screening agents with them. However, these particulate inorganic screening agents have the drawback of degrading the cosmeticity of cosmetic compositions, by generating a white film on the skin during application.

Applications FR 2882371, WO 2006/083326 and WO 98/37964 describe various processes for manufacturing composite particles constituted of a material comprising nanoparticles of metal oxides, such as titanium dioxide.

Application WO 2006/061835 describes compositions comprising spherical composites based on a metal oxide and on a hydrophobic polymer.

Known in the cosmetic field are application EP 1 388 550, which targets the use of composite particles comprising a core formed of a metal oxide coated with a silicone or fluoro compound and the use thereof as a photoprotective cosmetic composition, and application WO 98/22539, which describes a sunscreen containing a particle of silicon and/or of another solid compound in which the silicon is in stoichiometric excess, said particle having a mean diameter of less than 0.12 μm and being covered with a layer of oxide having a thickness ranging from 0.001 to 0.3 μm.

Anti-sun formulations that may contain as screening system spherical particles of composite material with a mean size of between 2 and 7 μm, $TiO_2$ encapsulated in spherical silica particles, are known, such as those sold under the name Eospoly TR by the company Creations Couleurs or under the name Sunsil T50 by Sunjin Chemical.

These screening materials have a better cosmeticity, but have the drawback of leading to formulations whose efficacy still remains insufficient.

There is thus still a need for anti-UV sun protection compositions based on inorganic UV-screening agents, which afford more efficient photoprotection and which do not have the drawbacks presented above.

Unexpectedly and advantageously, the inventors have shown that this need can be met by means of the compositions according to the present invention.

A first subject of the present invention relates to a composition containing, in a cosmetically acceptable medium, at least:

a) at least one aqueous phase and

b) composite particles A in spherical form, having a mean size of between 0.1 and 30 μm comprising at least one particulate UV-screening agent and a core constituted of at least one inorganic material and/or of at least one organic material, and

c) free particles B of inorganic UV-screening agent having a mean elementary size greater than 0.070 μm,

the content of composite particles A in the composition according to the invention ranges from 1% to 70% relative to the total weight of the cosmetic composition. the content of particles B in the composition according to the invention ranges from 0.1% and 40% relative to the total weight of the composition,
the core of the spherical composite particles A contains a material or a mixture of materials chosen from:

- $SiO_2$,
- poly(methyl methacrylate),
- copolymers of styrene and of a C1/C5 alkyl (meth)acrylate derivative,

the particulate UV-screening agent is inorganic and chosen from titanium oxides,
the particles B of inorganic UV-screening agent are metal oxides treated with at least one surface-treatment agent, in hydrophobically-modified coated form, chosen from :

- a dimethicone-treated titanium dioxide;
- a titanium dioxide treated with a silica/alumina/stearic acid or alumina/stearic acid mixture;
- an oily dispersion of $TiO_2$ particles treated with a mixture of stearic acid, polyhydroxystearic acid and alumina.

The composition according to the present invention is effective in photoprotection.

The following description and examples present other advantages, aspects and properties of the present invention.

## Definitions

The following definitions are used in the present text.

The compositions according to the present invention are photoprotective compositions intended to screen out UV radiation; these compositions are also known as anti-sun compositions or sun protection compositions.

The expression "cosmetically acceptable" means compatible with the skin and/or its appendages or mucous membranes, having a pleasant colour, odour and feel and not causing any unacceptable discomfort (stinging, tautness or redness) liable to discourage the consumer from using this composition.

The expression "mean size of the particles" is understood to mean the parameter D[4,3] measured using a "Mastersizer 2000" particle size analyser (Malvern). The light intensity scattered by the particles as a function of the angle at which they are lit is converted to size distribution according to Mie theory. The parameter D[4,3] is measured; this is the mean diameter of the sphere having the same volume as the particle. For a spherical particle, reference will often be made to the "mean diameter".

The expression "mean elementary size" means the size of non-aggregated particles.

The expression "free particles B" means that the particles B present in the composition are not integrated into the matrix and/or onto the surface of the matrix of the composite particles A.

The expression "particulate organic UV-screening agent" means any organic molecule which (1) is in the form of particles that are solid at 25°C and insoluble in the cosmetically acceptable medium of the composition of the invention, and which (2), via a mechanism known per se, by absorption and/or reflection and/or scattering of UVA and/or UVB radiation, make it possible to prevent or at least limit the contact of this radiation with human keratin materials.

The expression "human keratin materials" means the skin (body, face, area around the eyes), hair, eyelashes, eyebrows, body hair, nails, lips or mucous membranes.

## SCREENING COMPOSITE PARTICLES A

The spherical composite particles A used according to the invention preferably have a mean size of between 0.1 and 30 μm, preferably between 0.1 and 20 μm and even more preferentially between 0.1 and 10 μm.

The spherical particles A used according to the present invention comprise at least one particulate UV-screening agent and a core constituted of at least one inorganic material and/or of at least one organic material.

According to a first variant, the composite particles A contain a core comprising at least one organic material and/or at least one inorganic material, in which are included particles of particulate UV-screening agent. According to this embodiment, the matrix exhibits inclusions, and particles of particulate UV-screening agent are placed in the inclusions of the matrix.

According to a second variant, the spherical composite particles A contain a core made of an organic material and/or an inorganic material, covered with at least one layer of particulate UV-screening agent which may be connected to the matrix by means of a binder.

According to a third variant, the composite particles A contain a particulate UV-screening agent covered with at least one layer of an organic material and/or of an inorganic material.

The core may also be formed from one or more organic materials and/or inorganic materials. It may then be a continuous phase of materials such as an alloy, i.e. a continuous phase in which the materials can no longer be dissociable, or a discontinuous phase of materials, for example constituted of an organic or inorganic material covered with a layer of another different organic or inorganic material.

The weight content of particulate UV-screening agent in the composite particles A of the invention is preferably between 1% and 90%, preferably between 2% and 80% and even better still between 3% and 70%.

According to one variant, in particular when the composite particles A comprise a matrix covered with a layer of particulate UV-screening agent, the composite particles may furthermore be covered with an additional coating, in particular chosen from biodegradable or biocompatible materials, lipid materials, for instance surfactants or emulsifiers, polymers, and oxides.

The screening composite particles A are in spherical form.

The term "spherical" means that the particle has a sphericity index, i.e. the ratio between its largest diameter and its smallest diameter, of less than 1.2.

The content of composite particles A in the composition according to the invention ranges from 1% to 70%, preferably from 1.5% to 50% and preferably from 2% to 40% by weight relative to the total weight of the cosmetic composition.

The matrix of the spherical composite particle A contains a material or a mixture of materials chosen from:

- $SiO_2$,
- poly(methyl methacylate),
- copolymers of styrene and of a C1/C5 alkyl (meth)acrylate derivative.

The composite particles A in spherical form are characterized by a mean diameter of between 0.1 and 30 µm, preferably between 0.1 and 20 µm and more preferably between 0.1 and 10 µm.

The particulate UV-screening agents according to the invention are inorganic and are chosen from titanium oxides.

## PARTICULATE UV-SCREENING AGENTS

The particulate UV-screening agents are generally chosen from particulate organic UV-screening agents and inorganic screening agents.

### Inorganic screening agents

The inorganic UV-screening agent is generally chosen from metal oxides, preferably titanium, zinc or iron oxides, or mixtures thereof, and more particularly from titanium dioxide (amorphous or crystalline in rutile and/or anatase form), zinc oxide and mixtures thereof. Particularly preferably, the inorganic UV-screening agent is $TiO_2$.

These metal oxides may be in the form of particles, having a mean elementary size generally of less than 200 nm. Advantageously, the metal oxide particles used have a mean elementary size of less than or equal to 0.15 µm.

These metal oxides may also be in the form of layers, preferably multilayers with a mean thickness generally of less than 0.2 µm.

The inorganic UV-screening agents in accordance with the invention preferably have a mean elementary particle size of greater than 5 nm and less than 200 nm. According to one particularly preferred embodiment of the invention, this size preferably ranges from 10 nm to 150 nm.

According to one embodiment of the invention, the inorganic UV-screening agents may be titanium oxide-based nanopigments.

The inorganic UV-screening agents may be coated or uncoated.

The coated inorganic UV-screening agents are pigments that have undergone one or more surface treatments of chemical, electronic, mechanochemical and/or mechanical nature with compounds as described, for example, in Cosmetics & Toiletries, February 1990, Vol. 105, pp. 53-64, such as amino acids, beeswax, fatty acids, fatty alcohols, anionic surfactants, lecithins, sodium, potassium, zinc, iron or aluminium salts of fatty acids, metal alkoxides (titanium or aluminium alkoxides), polyethylene, silicones, proteins (collagen, elastin), alkanolamines, silicon oxides, metal oxides

or sodium hexametaphosphate.

As is known, silicones are organosilicon polymers or oligomers of linear or cyclic, branched or crosslinked structure, of variable molecular weight, obtained by polymerization and/or polycondensation of suitably functionalized silanes, and are essentially constituted of a repetition of main units in which the silicon atoms are linked together via oxygen atoms (siloxane bond), optionally substituted hydrocarbon-based radicals being directly attached to said silicon atoms via a carbon atom.

The term "silicones" also encompasses the silanes required for their preparation, in particular alkylsilanes.

The silicones used for coating the pigments that are suitable for the present invention are preferably chosen from the group containing alkylsilanes, polydialkylsiloxanes and polyalkylhydrogenosiloxanes. Even more preferentially, the silicones are chosen from the group containing octyltrimethylsilane, polydimethylsiloxanes and polymethylhydrogenosiloxanes.

Of course, before being treated with silicones, the metal oxide pigments may have been treated with other surface agents, in particular with cerium oxide, alumina, silica, aluminium compounds or silicon compounds, or mixtures thereof.

The coated pigments are more particularly titanium oxides that have been coated:

- with silica, such as the product Sunveil from the company Ikeda,
- with silica and iron oxide, such as the product Sunveil F from the company Ikeda,
- with silica and alumina, such as the products Microtitanium Dioxide MT 500 SA and Microtitanium Dioxide MT 100 SA from the company Tayca and Tioveil from the company Tioxide,
- with alumina, such as the products Tipaque TTO-55 (B) and Tipaque TTO-55 (A) from the company Ishihara and UVT 14/4 from the company Kemira,
- with alumina and aluminium stearate, such as the products Microtitanium Dioxide MT 100 T, MT 100 TX, MT 100 Z and MT-01 from the company Tayca, the products Solaveil CT-10 W and Solaveil CT 100 from the company Uniqema and the product Eusolex T-AVO from the company Merck,
- with silica, alumina and alginic acid, such as the product MT-100 AQ from the company Tayca,
- with alumina and aluminium laurate, such as the product Microtitanium Dioxide MT 100 S from the company Tayca,
- with iron oxide and iron stearate, such as the product Microtitanium Dioxide MT 100 F from the company Tayca,
- with zinc oxide and zinc stearate, such as the product BR 351 from the company Tayca,
- with silica and alumina and treated with a silicone, such as the products Microtitanium Dioxide MT 600 SAS, Microtitanium Dioxide MT 500 SAS or Microtitanium Dioxide MT 100 SAS from the company Tayca,
- with silica, alumina and aluminium stearate and treated with a silicone, such as the product STT-30-DS from the company Titan Kogyo,
- with silica and treated with a silicone, such as the product UV-Titan X 195 from the company Kemira,
- with alumina and treated with a silicone, such as the products Tipaque TTO-55 (S) from the company Ishihara or UV Titan M 262 from the company Kemira,
- with triethanolamine, such as the product STT-65-S from the company Titan Kogyo,
- with stearic acid, such as the product Tipaque TTO-55 (C) from the company Ishihara,
- with sodium hexametaphosphate, such as the product Microtitanium Dioxide MT 150 W from the company Tayca.
- $TiO_2$ treated with octyltrimethylsilane, sold under the trade name T 805 by the company Degussa Silices,
- $TiO_2$ treated with a polydimethylsiloxane, sold under the trade name 70250 Cardre UF TiO2SI3 by the company Cardre,
- anatase/rutile $TiO_2$ treated with a polydimethylhydrogenosiloxane, sold under the trade name Microtitanium Dioxide USP Grade Hydrophobic by the company Color Techniques.

The uncoated titanium oxide pigments are sold, for example, by the company Tayca under the trade names Microtitanium Dioxide MT 500 B or Microtitanium Dioxide MT 600 B, by the company Degussa under the name P 25, by the company Wackherr under the name Transparent titanium oxide PW, by the company Miyoshi Kasei under the name UFTR, by the company Tomen under the name ITS and by the company Tioxide under the name Tioveil AQ.

The uncoated zinc oxide pigments are for example:

- those sold under the name Z-Cote by the company Sunsmart;
- those sold under the name Nanox by the company Elementis;
- those sold under the name Nanogard WCD 2025 by the company Nanophase Technologies.

The coated zinc oxide pigments are for example:

- those sold under the name Zinc Oxide CS-5 by the company Toshibi (ZnO coated with polymethylhydrogenosiloxane);

- those sold under the name Nanogard Zinc Oxide FN by the company Nanophase Technologies (as a 40% dispersion in Finsolv TN, $C_{12}$-$C_{15}$ alkyl benzoate);
- those sold under the name Daitopersion ZN-30 and Daitopersion ZN-50 by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO by the company Daikin (ZnO coated with perfluoroalkyl phosphate and copolymer based on perfluoroalkylethyl as a dispersion in cyclopentasiloxane);
- those sold under the name SPD-Z1 by the company Shin-Etsu (ZnO coated with silicone-grafted acrylic polymer, dispersed in cyclodimethylsiloxane);
- those sold under the name Escalol Z100 by the company ISP (alumina-treated ZnO dispersed in an ethylhexyl methoxycinnamate/PVP-hexadecene copolymer/methicone mixture);
- those sold under the name Fuji ZnO-SMS-10 by the company Fuji Pigment (ZnO coated with silica and polymethylsilsesquioxane);
- those sold under the name Nanox Gel TN by the company Elementis (ZnO dispersed at a concentration of 55% in C12-C15 alkyl benzoate with hydroxystearic acid polycondensate).

The uncoated cerium oxide pigments may be, for example, those sold under the name Colloidal Cerium Oxide by the company Rhone-Poulenc.

The uncoated iron oxide pigments are, for example, sold by the company Arnaud under the names Nanogard WCD 2002 (FE 45B), Nanogard Iron FE 45 BL AQ, Nanogard FE 45R AQ and Nanogard WCD 2006 (FE 45R) or by the company Mitsubishi under the name TY-220.

The coated iron oxide pigments are sold, for example, by the company Arnaud under the names Nanogard WCD 2008 (FE 45B FN), Nanogard WCD 2009 (FE 45B 556), Nanogard FE 45 BL 345 and Nanogard FE 45 BL or by the company BASF under the name Transparent Iron Oxide.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A, and also the mixture of titanium dioxide and zinc dioxide coated with alumina, silica and silicone, such as the product M 261 sold by the company Kemira, or coated with alumina, silica and glycerol, such as the product M 211 sold by the company Kemira.

According to the invention, coated or uncoated titanium oxide pigments are particularly preferred.

The titanium oxide may be in rutile and/or anatase form and/or in an amorphous or substantially amorphous form.

1) Particulate organic screening agents

The particulate organic UV-screening agent(s) comprising at least one UV radiation-absorbing group can be chosen in particular from organic insoluble UV-screening agents of oxalanilide, triazine, benzotriazole, vinylamide, cinnamide, benzazole, benzofuran, arylvinylidene-ketone, acrylonitrile amide, acrylonitrile sulfonamide, acrylonitrile carbamate or phenylenebisbenzoxazinone type.

Among the particulate organic UV-screening agents of oxalanilide type, mention may be made of those corresponding to the formula:

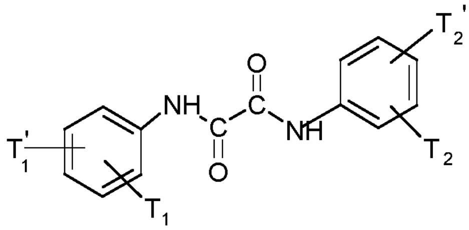 (I)

in which $T_1$, $T_1$', $T_2$ and $T_2$', each independently represent a $C_1$-$C_8$ alkyl radical or a $C_1$-$C_8$ alkoxy radical. These compounds are described in patent application WO 95/22959. By way of examples, mention may be made of the commercial products Tinuvin® 315 and Tinuvin® 312 sold by the company Ciba-Geigy corresponding respectively to the formulae:

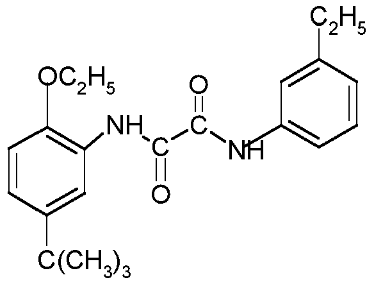

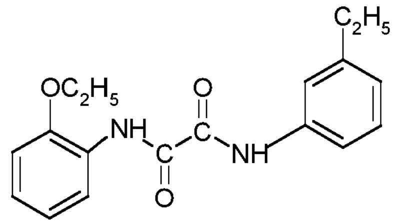

The particulate organic UV-screening agents of triazine type correspond to the following general formula:

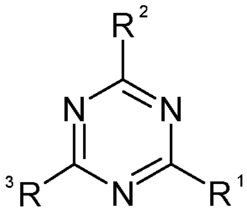

(II)

in which $R_1$, $R_2$ and $R_3$ each independently represent a phenyl, phenoxy or pyrrolo group, these groups being unsubstituted or each independently bearing one, two or three substituents chosen from -OH, $C_{1}$-$_{18}$ alkyl, $C_1$-$C_{18}$ alkoxy, carboxy($C_1$-$C_{18}$ alkyl), $C_5$-$C_8$ cycloalkyl, methylbenzylidenecamphor, -(CH=CR')n(CO)-$OR_4$ where R' represents a hydrogen atom, a cyano group or a $COOR_4$ group, with $R_4$ = $C_1$-$C_{18}$ alkyl or cinnamyl, and n is 0 or 1.

These compounds are described in WO 97/03643, GB 2286774, EP 743309, WO 98/22447, GB 2319523 and EP-A-0 790 243.

Mention will be made more particularly of the following compounds:

- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl $\alpha$-cyano-4-aminocinnamate)-s-triazine.

Among the particulate organic UV-screening agents of triazine type that can be used for the present invention, mention may also be made of the insoluble derivatives of s-triazine, bearing benzotriazole and/or benzothizole groups such as those described in application WO 98/25922.

Among these compounds, mention may more particularly be made of 2,4,6-tris[(3'-benzotriazol-2-yl-2'-hydroxy-5'-methyl)phenylamino]-s-triazine and 2,4,6-tris[(3'-benzotriazol-2-yl-2'-hydroxy-5'-tert-octyl)phenylamino]-s-triazine.

Among the particulate organic UV-screening agents of benzotriazole type, mention may be made of those of formula (III) below, described for example in international application WO 95/22959:

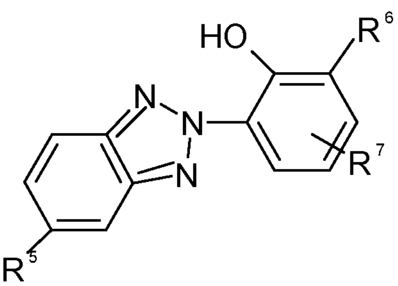

(III)

in which $R_5$ denotes a hydrogen atom or a $C_1$-$C_{18}$ alkyl radical, $R_6$ and $R_7$, which may be identical or different, each independently denote a $C_1$-$C_{18}$ alkyl radical optionally substituted with a phenyl group.

By way of examples of compounds of formula (III), mention may be made of the commercial products Tinuvin® 328, 320, 234 and 350 from the company Ciba-Geigy corresponding respectively to the following formulae:

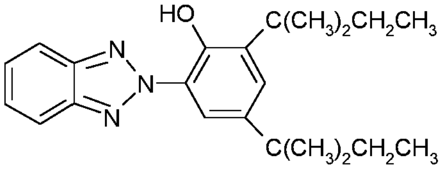

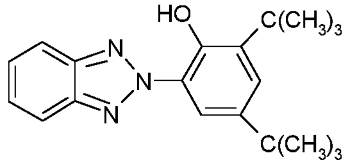

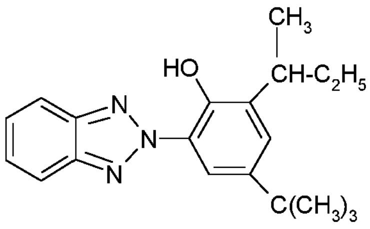

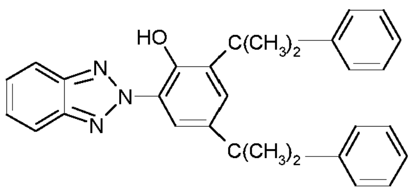

and the particulate screening agents of polytriazine type as mentioned in patent FR 2 861 075.

Among the particulate organic UV-screening agents of benzotriazole type, mention may also be made of the compounds described in patents US 5687521, US 5687521, US 5373037 and US 5362881, and among them in particular the [2,4'-dihydroxy-3-(2H-benzotriazol-2-yl)-5-(1,1,3,3-tetramethylbutyl)-2'-n-octoxy-5'-benzoyl]diphenylmethane of formula:

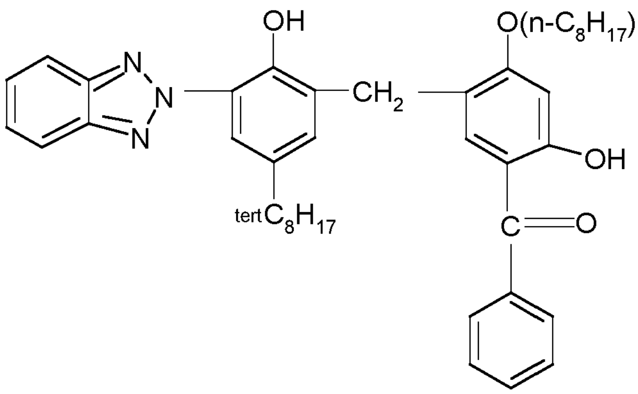

sold under the name Mixxim® PB30 by the company Fairmount Chemical.

Other particulate organic UV-screening agents of benzotriazole type are the methylenebis(hydroxyphenylbenzotriazole) derivatives having the following structure:

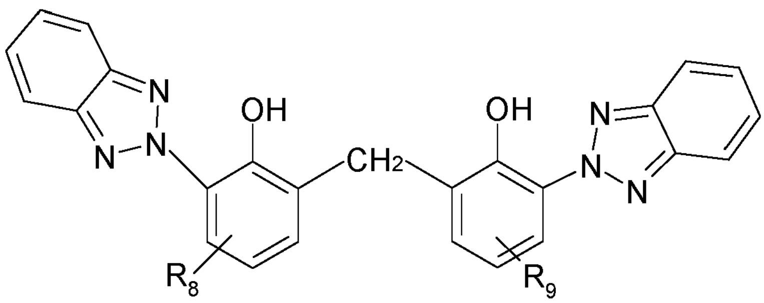
(IV)

in which $R_8$ and $R_9$, which may be identical or different, each represent a $C_1$-$C_{18}$ alkyl radical possibly substituted with one or more radicals chosen from $C_1$-$C_4$ alkyl and $C_5$-$C_{12}$ cycloalkyl, or aryl. These compounds are known and described in applications US 5237071, US 5166355, GB-A-2 303549, DE 19726184 and EP-A-893119.

In formula (IV) defined above, the $C_1$-$C_{18}$ alkyl groups may be linear or branched and are, for example, methyl, ethyl, n-propyl, isopropyl, n-butyl, isobutyl, tert-butyl, tert-octyl, n-amyl, n-hexyl, n-heptyl, n-octyl, isooctyl, n-nonyl, n-decyl, n-undecyl, n-dodecyl, tetradecyl, hexydecyl or octadecyl; the $C_5$-$C_{12}$ cyloalkyl groups are, for example, cyclopentyl, cyclohexyl or cyclooctyl; the aryl groups are, for example, phenyl or benzyl.

Among the compounds of formulae (IV), preference is more particularly given to those having the following structure:

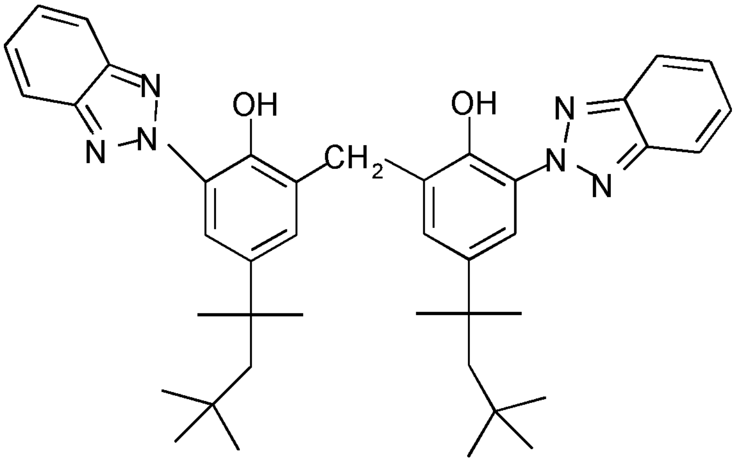

compound (a)

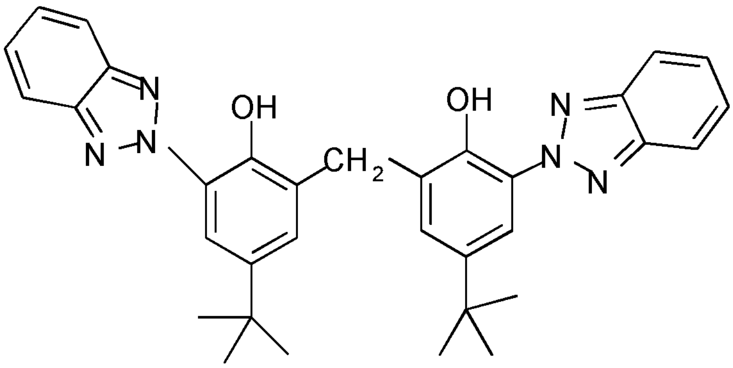

compound (b)

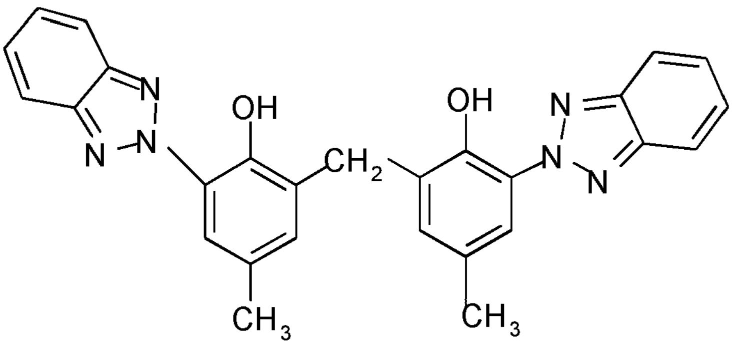

compound (c)

The compound (a) with the nomenclature 2,2'-methylenebis[6-(2H-benzotriazol-2-yl)-4-(1,1,3,3-tetramethylbutyl)phenol] is sold in non-micronized form under the name Mixxim® BB/100 by the company Fairmount Chemical and in micronized form under the name Tinosorb® M by the company Ciba Specialty Chemicals.

The compound (c) with the nomenclature 2,2'-methylenebis[6-(2H-benzotriazol-2-yl)-4-(methyl)phenol] is sold in solid form under the name Mixxim® BB/200 by the company Fairmount Chemical.

Among the particulate organic screening agents of the vinylamide type, mention may, for example, be made of the compounds of formula (V) which are described in application WO 95/22959:

$$T_3\text{-}(Y)_r\text{-}C(=O)\text{-}C(T_4)=C(T_5)\text{-}N(T_6)(T_7) \qquad (V)$$

in which $T_3$ is a $C_1$-$C_{18}$, preferably $C_1$-$C_5$, alkyl radical or a phenyl group optionally substituted with one, two or three radicals chosen from OH, $C_1$-$C_{18}$ alkyl, $C_1$-$C_8$ alkoxy or -C(=O)-O$T_8$ where $T_8$ represents a $C_1$-$C_{18}$ alkyl group; $T_4$, $T_5$, $T_6$ and $T_7$ each independently represent a $C_1$-$C_{18}$, preferably $C_1$-$C_5$ alkyl radical, or a hydrogen atom; Y represents an -NH- group or an oxygen atom and r is 0 or 1.

Among these compounds, mention will more particularly be made of:

4-octylam ino-3-penten-2-one;
ethyl 3-octylamino-2-butenoate;
3-octylamino-1-phenyl-2-buten-1-one and
3-dodecylamino-1-phenyl-2-buten-1-one.

Among the particulate organic screening agents of cinnamide type, mention may be made of the compounds such as those described in application WO 95/22959 and corresponding to the following formula:

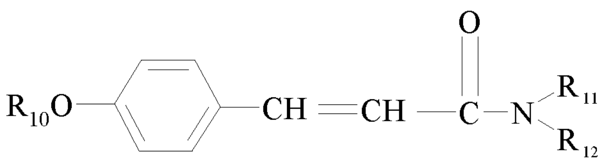

(VI)

in which:

$R_{10}$ represents a hydrogen atom or a $C_1$-$C_4$ alkyl group, preferably methyl or ethyl,
$R_{11}$ represents a hydrogen atom or a $C_1$-$C_4$ alkyl group, preferably methyl or ethyl,
$R_{12}$ represents a -(CONH)s-phenyl group where s is 0 or 1 and the phenyl group can be substituted with one, two or three groups chosen from OH, $C_1$-$C_{18}$ alkyl, $C_1$-$C_8$ alkoxy or -C(=O)-O$R_{13}$ where $R_{13}$ is a $C_1$-$C_{18}$ alkyl, and more preferentially $R_{12}$ represents a phenyl, 4-methoxyphenyl or phenylaminocarbonyl group.

Mention may also be made of the bis[alpha, beta-disubstituted cinnamide] dimers described, for example, in patent US 5 888 481, having the structure:

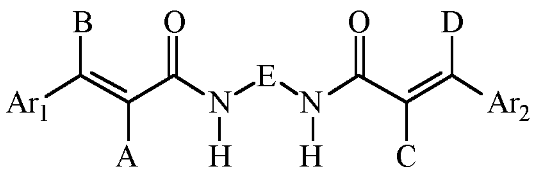

(VII)

in which:

$Ar_1$ and $Ar_2$, which may be identical or different, each represent a phenyl radical, an aromatic heterocycle, a group comprising a condensed phenyl nucleus or a group comprising a condensed aromatic heterocycle, and can bear one or more identical or different substituents,
B and D, which other than a hydrogen atom, each independently represent an organic radical,
A and C each independently represent an organic radical, and
E represents a divalent organic radical,
with the exclusion of the compounds for which $Ar_1$ and $Ar_2$ both represent a phenyl group bearing a substituent -OR where R represents a hydrogen atom or organic radical, A and C both represent a cyano group, B and D both represent a $C_1$-$C_{35}$ alkyl or alkynyl group, and E represents a divalent organic radical,
and in particular the compound having the structure:

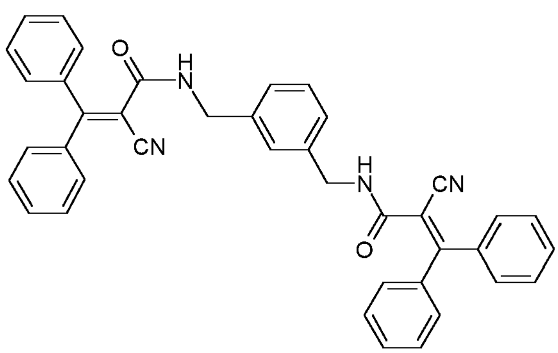

Among the particulate organic screening agents of the benzazole type, mention may be made of those corresponding to one of the following formulae:

(VIII) 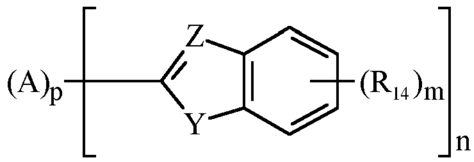

(IX) 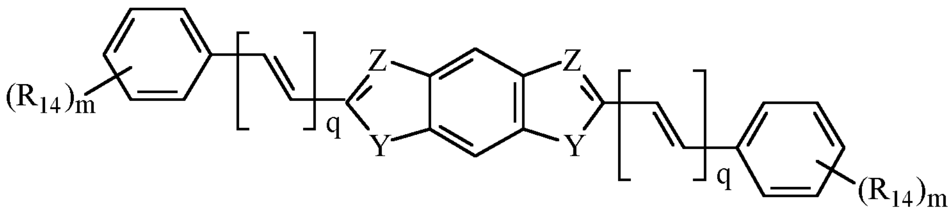

(X) 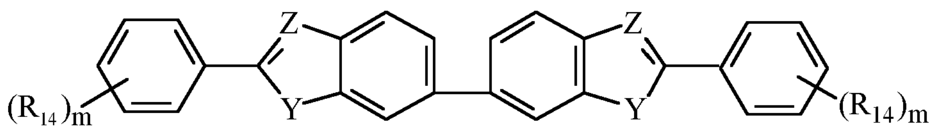

in which:

each of the symbols Y independently represents an oxygen or sulfur atom or an $NR_{15}$ group,
each of the symbols Z independently represents a nitrogen atom or a CH group, each of the symbols $R_{14}$ independently represents an OH group, a halogen atom, a linear or branched $C_1$-$C_8$alkyl group optionally containing a silicon atom, or a linear or branched $C_1$-$C_8$alkoxy group,
each of the numbers m is independently 0, 1 or 2,
n represents an integer between 1 and 4, inclusive,
p is equal to 0 or 1,
each of the numbers q is independently equal to 0 or 1,
each of the symbols $R_{15}$ independently represents a hydrogen atom, a benzyl group or a linear or branched $C_1$-$C_8$ alkyl group optionally containing a silicon atom,
A represents a radical of valency n chosen from those having the formulae:

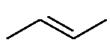
(a)

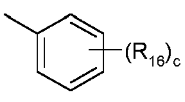
(b)

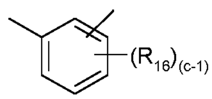
(c)

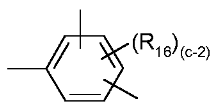
(d)

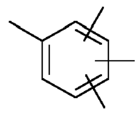
(e)

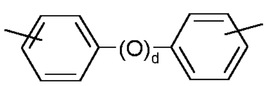
(f)

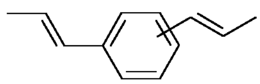
(g)

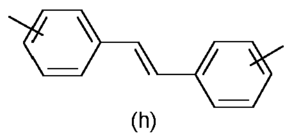
(h)

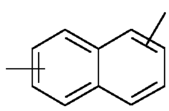
(i)

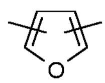
(j)

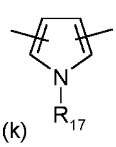
(k)

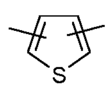
(l)

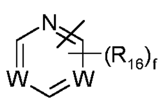
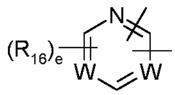
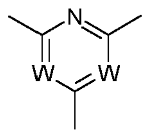

(m) (n) (o)

in which each of the symbols $R_{16}$ independently represents a halogen atom, a linear or branched $C_1$-$C_4$ alkyl or alkoxy group, or a hydroxyl group,

$R_{17}$ represents a hydrogen atom or a linear or branched $C_1$-$C_4$ alkyl group, c = 0 - 4, d = 0 - 3, e = 0 or 1 and f = 0 - 2.

These compounds are in particular described in patents DE 676 103 and CH 350763, patent US 5501850, patent US 5961960, patent application EP 0 669 323, patent US 5518713, patent US 2463264, the article in J. Am. Chem. Soc., 79, 5706 - 5708, 1957, the article published in J. Am. Chem. Soc., 82, 609 - 611, 1960, patent application EP 0 921 126 and patent application EP 0712855. By way of examples of preferred compounds of formula (VIII) of the 2-arylbenzazole family, mention may be made of 2-benzoxazol-2-yl-4-methylphenol, 2-(1H-benzimidazol-2-yl)-4-methoxyphenol or 2-benzothiazol-2-ylphenol, it being possible for these compounds to be prepared, for example, according to the processes described in patent CH 350 763.

By way of examples of preferred compounds of formula (VIII) of the benzimidazolylbenzazole family, mention will be made of 2,2'-bis-benzimidazole, 5,5',6,6'-tetramethyl-2,2'-bisbenzimidazole, 5,5'-dimethyl-2,2'-bisbenzimidazole, 6-methoxy-2,2'-bisbenzimidazole, 2-(1H-benzimidazol-2-yl)benzothiazole, 2-(1H-benzimidazol-2-yl)benzoxazole and N,N'-dimethyl-2,2'-bisbenzimidazole, it being possible for these compounds to be prepared according to the procedures described in patents US 5 961 960 and US 2 463 264.

By way of examples of preferred compounds of formula (VIII) of the phenylenebenzazole family, mention will be made of 1,4-phenylenebis(2-benzoxazole), 1,4-phenylenebis(2-benzimidazole), 1,3-phenylenebis(2-benzoxazole), 1,2-phenylenebis(2-benzoxazole), 1,2-phenylenebis(benzimidazole), 1,4-phenylenebis(N-2-ethylhexyl-2-benzimidazole) and 1,4-phenylenebis(N-trimethylsilylmethyl-2-benzimidazole), it being possible for these compounds to be prepared according to the procedures described in patent US 2 463 264 and in the publications J. Am.Chem. Soc., 82, 609 (1960) and J. Am. Chem. Soc., 79, 5706 - 5708 (1957).

By way of examples of preferred compounds of formula (VIII) of the benzofuranylbenzoxazole family, mention will be made of 2-(2-benzofuranyl)benzoxazole, 2-(benzofuranyl)-5-methylbenzoxazole and 2-(3-methyl-2-benzofuranyl)benzoxazole, it being possible for these compounds to be prepared according to the procedures described in patent US 5 518 713.

As preferred compounds of formula (IX), mention may be made, for example, of 2,6-diphenyl-1,7-dihydrobenzo[1,2-d;4,5-d']diimidazole corresponding to the formula:

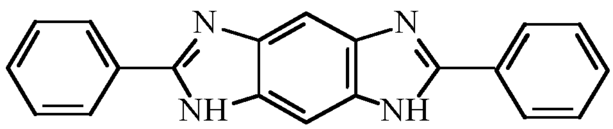

or 2,6-distyryl-1,7-dihydrobenzo[1,2-d; 4,5-d']diimidazole or else 2,6-di(p-tert-butylstyryl)-1,7-dihydrobenzo[1,2-d; 4,5-d']diimidazole, which can be prepared according to the processes described in application EP 0 669 323.

As preferred compound of formula (X), mention may be made of 5,5'-bis-[(phenyl-2)benzimidazole] having the formula:

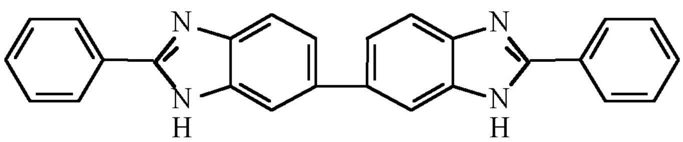

the preparation of which is described in J. Chim. Phys., 64, 1602 (1967).

Among these UV radiation-screening organic insoluble compounds, 2-(1H-benzimidazol-2-yl)benzoxazole, 6-methoxy-2,2'-bisbenzimidazole, 2-(1H-benzimidazol-2-yl)benzothiazole, 1,4-phenylenebis(2-benzoxazole), 1,4-phenylenebis(2-benzimidazole), 1,3-phenylenebis(2-benzoxazole), 1,2-phenylenebis(2-benzoxazole), 1,2-phenylenebis(2-benzimidazole) and 1,4-phenylenebis(N-trimethylsilylmethyl-2-benzimidazole) are quite particularly preferred.

Another family of particulate screening agents is the family of arylvinylene-ketones chosen from those corresponding to one of the following formulae (XI) and (XII):

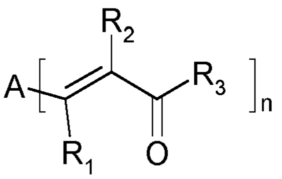

(XI)

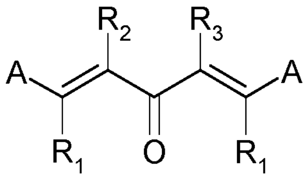

(XII)

in which:

n = 1 or 2,
A, in formula (XI) when n = 1 or in formula (XII), is an aryl radical chosen from the following formulae (a) to (d) or in formula (XI) when n = 2, is a radical chosen from the following formulae (e) to (h):

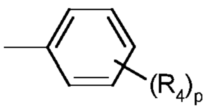

(a)

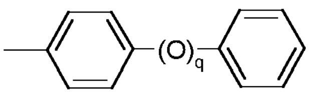

(b)

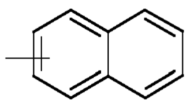

(c)

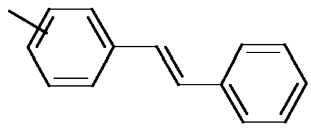

(d)

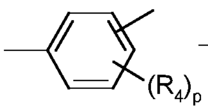

(e)

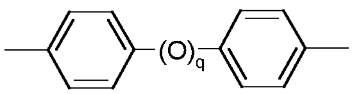

(f)

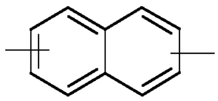

(g)

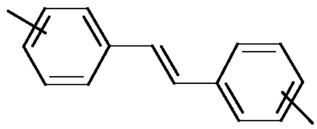

(h)

in which:

each of the symbols $R_4$ independently represents an OH group, a halogen atom, a linear or branched $C_1$-$C_6$alkyl group optionally containing a silicon atom, a linear or branched $C_1$-$C_6$alkoxy group optionally containing a silicon atom, a linear or branched $C_1$-$C_5$alkoxycarbonyl group, or a linear or branched $C_1$-$C_6$alkylsulfonamide group optionally containing a silicon atom or an amino acid function,
p represents an integer between 0 and 4, inclusive,
q represents 0 or 1,
$R_1$ represents hydrogen or an OH group,
$R_2$ represents hydrogen, a linear or branched $C_1$-$C_6$,alkyl group optionally containing a silicon atom, a cyano group, a $C_1$-$C_6$ alkylsulfonyl group or phenylsulfonyl group,
$R_3$ represents a linear or branched $C_1$-$C_6$ alkyl group optionally containing a silicon atom or a phenyl group which can form a bicycle and which is optionally substituted with one or two $R_4$ radicals,
or $R_2$ and $R_3$ together form a monocyclic, bicyclic or tricyclic $C_2$-$C_{10}$ hydrocarbon-based residue, optionally interrupted with one or more nitrogen, sulfur and oxygen atoms and which can contain another carbonyl, and optionally substituted with a linear or branched $C_1$-$C_8$ alkylsulfonamide group optionally containing a silicon atom or an amino acid function; on the condition that, when n=1, $R_2$ and $R_3$ do not form a camphor nucleus.

By way of examples of UV radiation-screening insoluble compounds of formula (XI) in which n=1, mention may be made of the following families:

styryl ketone (Kao JP 04 134 042) such as 1-(3,4-dimethoxyphenyl)-4,4-dimethylpent-1-en-3-one:

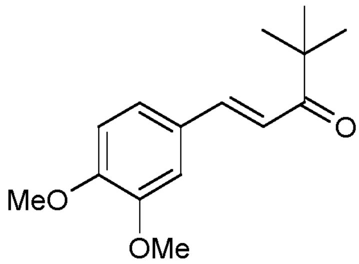

benzylidenecineole (E. Mariani et al, 16th IFSCC Congress, New York (1990)) such as 1,3,3-trimethyl-5-(4-methoxybenzylidene)-2-oxabicyclo[2.2.2]octan-6-one:

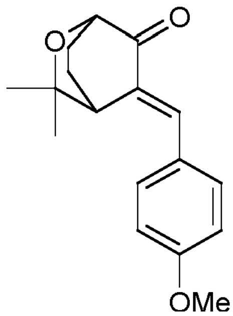

benzylidenechromanone (Kao JP 04 134 043) such as 3-(4-methoxybenzylidene)-2,3,4a,8a-tetrahydrochromen-4-one:

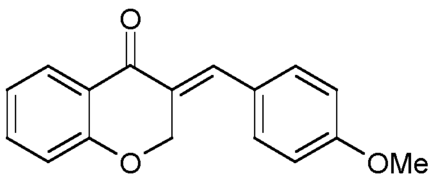

benzylidenethiochromanone (Kao JP 04 134 043) such as 3-(4-methoxybenzylidene-2,3,4a,8a-tetrahdrochromene-4-thione:

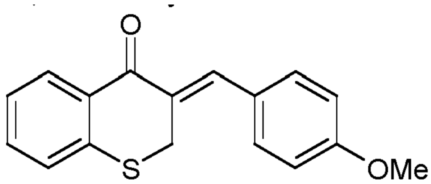

benzylidenequinuclidinone (Merck EP 0 576 974) such as 4-methoxybenzylidene-1-azabicyclo[2.2.2]octan-3-one:

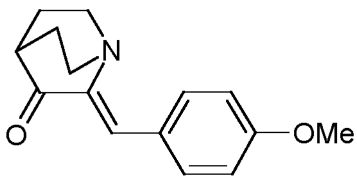

benzylidenecycloalkanone (Henkel FR 2 395 023) such as 2-(4-methoxybenzylidene)cyclopentanone and 2-(4-methoxybenzylidene)cyclohexanone:

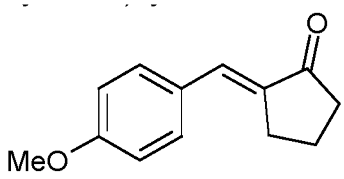
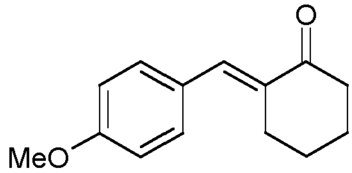

benzylidenehydantoin (Ajinomoto JP 01 158 090) such as 5-(3,4-dimethoxybenzylidene)imidazolidine-2,4-dione:

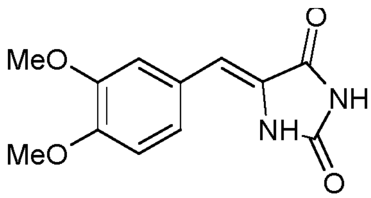

benzylideneindanone (Kao JP 04 134 043) such as 2-(4-methoxybenzylidene)indan-1-one:

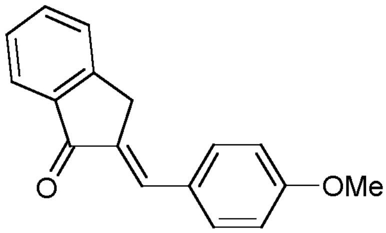

benzylidenetetralone (Kao JP 04 134 043) such as 2-(4-methoxybenzylidene)-3,4-dihydro-2H-naphthalen-1-one:

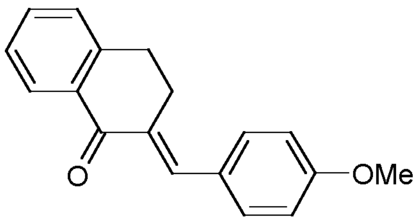

benzylidenefuranone (L'Oréal EP 0 390 683) such as 4-(4-methoxybenzylidene)-2,2,5,5-tetramethyldihydrofuran-3-one:

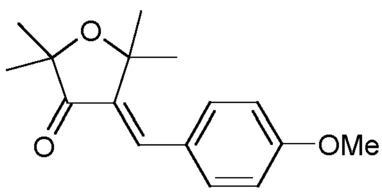

benzylidenebenzofuranone (Kao JP 04 134 041) such as 2-benzylidenebenzofuran-3-one:

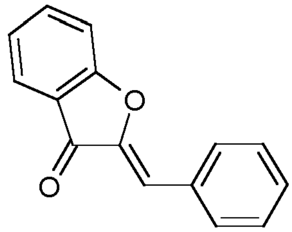

benzylideneindanedione such as 2-(3,5-di(tert-butyl)-4-hydroxybenzylidene)indane-1,3-dione:

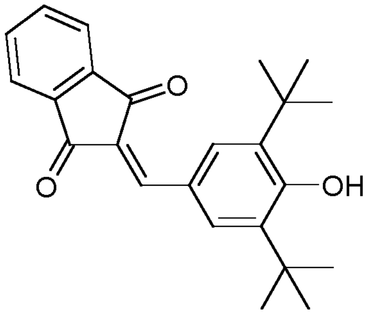

benzylidenebenzothiofuranone (Kao JP 04,134,043) such as 2-benzylidenebenzo[b]thiophen-3-one:

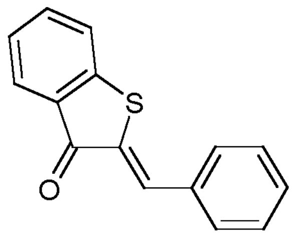

benzylidenebarbituric such as 5-(4-methoxybenzylidene)-1,3-dimethylpyrimidine-2,4,6-trione:

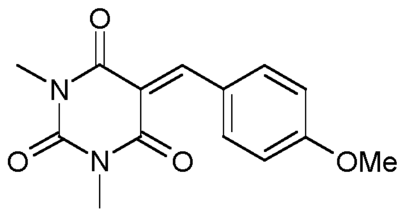

benzylidenepyrazolone such as 4-(4-methoxybenzylidene)-5-methyl-2-phenyl-2,4-dihydropyrazol-3-one:

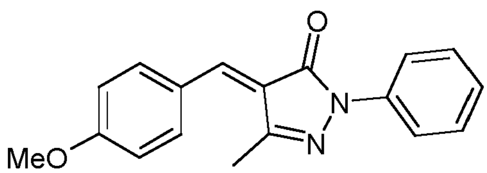

benzylideneimidazolone such as 5-(4-methoxybenzylidene)-2-phenyl-3,5-dihydroimidazol-4-one:

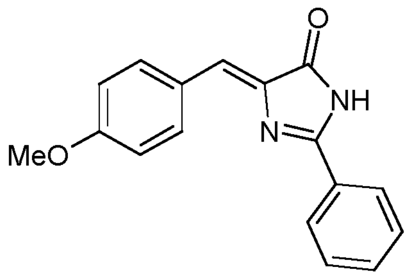

chalcone such as 1-(2-hydroxy-4-methoxyphenyl)-3-phenylpropenone:

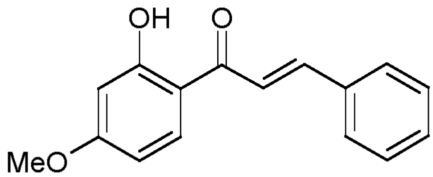

benzylidenone (screening tautomeric form of dibenzoylmethanes; L'Oréal FR 2 506 156) such as 3-hydroxy-1-(2-hydroxy-4-methoxyphenyl)-3-phenylpropenone:

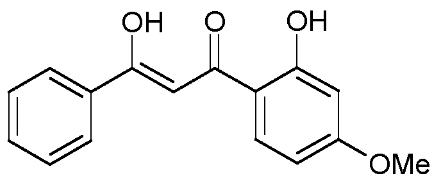

By way of examples of UV radiation-screening insoluble compounds of formula (XI) in which n=2, mention may be made of the following families:

phenylenebismethylidenenorcamphor (Merck EP 0 693 471) such as 1,4-phenylenebis{3-methylidenebicyclo[2.2.1]heptan-2-one}:

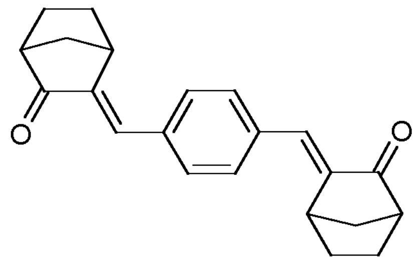

phenylenebismethylidenecamphor (L'Oréal FR 2 528 420) such as 1,4-phenylenebis{3-methylidene-1,7,7-trimethylbicyclo[2.2.1]heptan-2-one}:

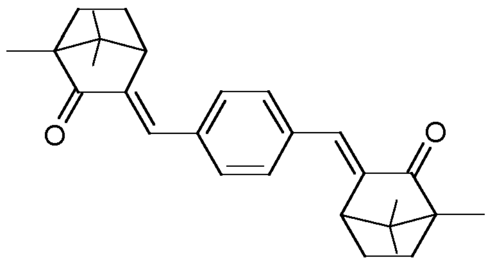

or 1 ,3-phenylenebis{3-methylidene-1,7,7-trimethylbicyclo[2.2.1]heptan-2-one}:

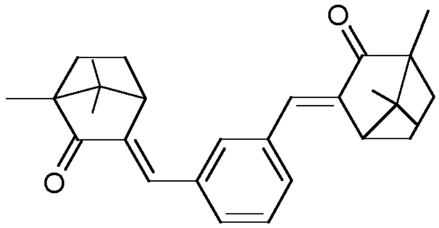

phenylenebismethylidenecamphorsulfonamide (L'Oréal FR 2 529 887) such as 1,4-phenylenebis{3,3'-methylidenecamphor-10,10'-ethylsulfonamide or -(2-ethylhexyl)sulfonamide}:

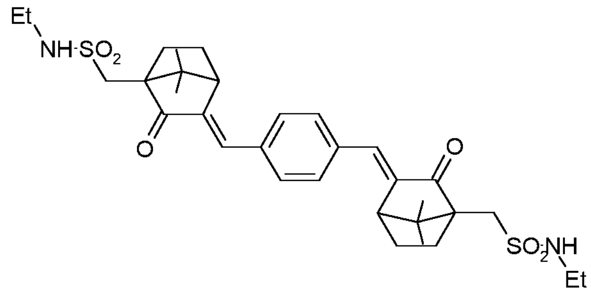

or

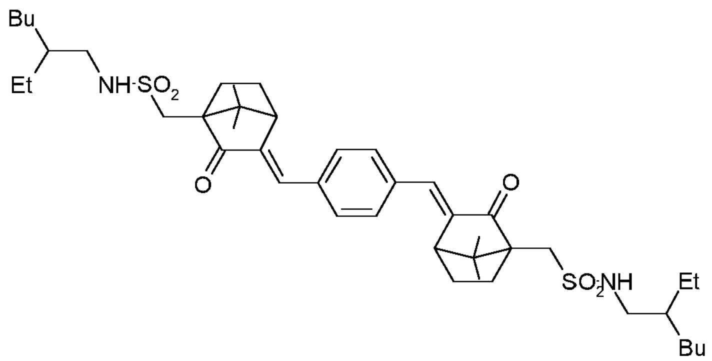

phenylenebismethylidenecineole (E. Mariani et al, 16th IFSCC Congress, New York (1990)) such as 1,4-phenylenebis{5-methylidène-3,3-dimethyl-2-oxa-bicyclo[2.2.2]octan-6-one}:

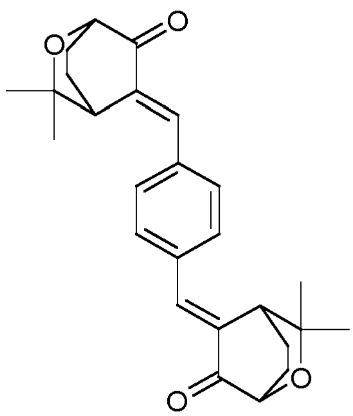

phenylenebis(methylideneketotricyclodecane) (Merck EP 0 694 521) such as 1,4-phenylenebis(octahydro-4,7-methano-6-inden-5-one):

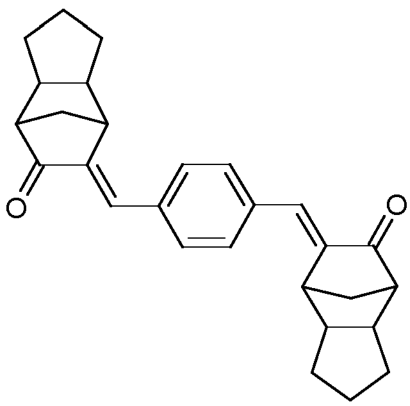

phenylenebis(alkylene ketone) (Kao JP 04 134 041) such as 1,4-phenylenebis(4,4-dimethylpent-1-en-3-one):

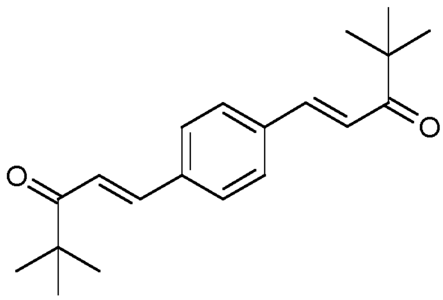

phenylenebis(methylidenefuranone) (L'Oréal FR 2 638 354) such as 1,4-phenylenebis(4-methylidene-2,2,5,5-tetramethyldihydrofuran-3-one):

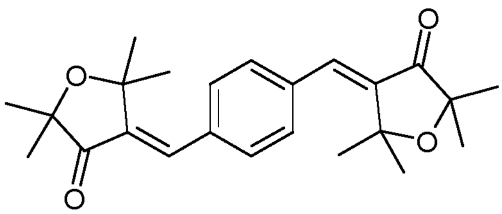

phenylenebis(methylidenequinuclidinone) (Merck EP 0 714 880) such as 1,4-phenylenebis{2-methylidene-1-azabicyclo[2.2.2]octan-3-one}:

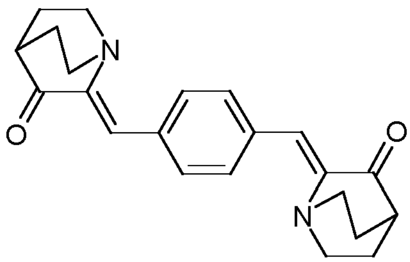

As compounds of formula (XII), mention may be made of the following families:

- bisbenzylidenecycloalkanone such as 2,5-dibenzylidenecyclopentanone:

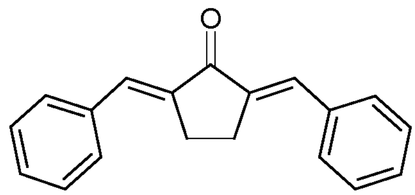

gamma-pyrone (Kao JP 04 290 882) such as 2,6-bis(3,4-dimethoxyphenyl)pyran-4-one:

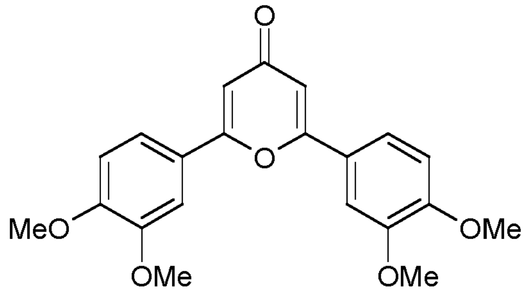

Another family of particulate screening agents that can be used in the present invention are the acrylonitrile amide, sulfonamide and carbamate derivatives corresponding to the following formula:

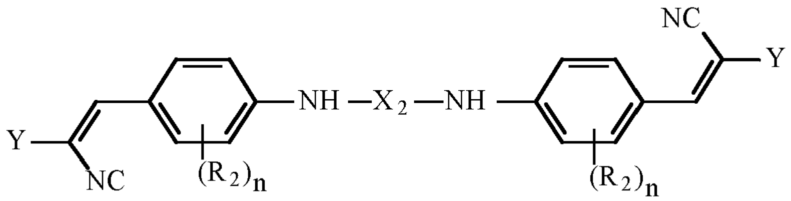

(XIII)

in which:

$X_2$ represents a divalent radical of formula -(C=O)-R'$_3$-(C=O)- , -$SO_2$-R"$_3$-$SO_2$- or -(C=O)-O-R"$_3$-O-(C=O)-,
Y represents a -(C=O)-$R_4$ or -$SO_2R_6$ radical,
$R_2$ represents a linear or branched $C_1$-$C_8$ alkyl group,
n is 0, 1 or 2,
R'$_3$ represents a single bond or R"3,
R"$_3$ represents a linear or branched divalent $C_1$-$C_{30}$ alkylene or $C_3$-$C_{30}$ alkenylene radical, which may bear one or more hydroxyl substituents and which may contain in the carbon-based chain one or more heteroatoms chosen from oxygen, nitrogen and silicon atoms,
$R_4$ represents an -$OR_6$ or -$NHR_6$ radical,
$R_5$ represents a linear or branched $C_1$-$C_{30}$ alkyl radical, or a phenyl nucleus which may be substituted with $C_1$-$C_4$ alkyl or alkoxy radicals,
$R_6$ represents a linear or branched $C_1$-$C_{30}$ alkyl or $C_3$-$C_{30}$ alkenyl radical, which may bear one or more hydroxyl substituents and which may contain in the carbon-based chain one or more heteroatoms chosen from oxygen, nitrogen and silicon atoms.

Although in formula (XIII) above only the isomers in which the cyano substituent is in the cis position with respect to the para-aminophenyl substituent are represented, this formula should be understood as also encompassing the corresponding trans isomers for each of the two double bonds and, independently, the cyano and para-aminophenyl substituents can be in the cis or trans configuration with respect to one another.

Another family of particulate organic screening agents that can be used according to the present invention is formed by the phenylenebis(benzoxazinone) derivatives having the formula:

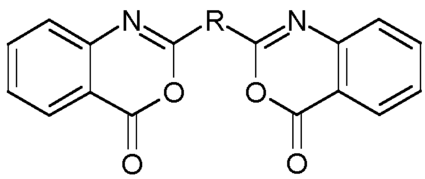

(XIV)

in which R represents a divalent aromatic residue chosen from the following formulae (e) to (h):

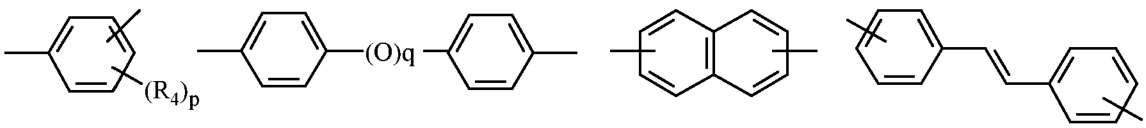

(e) (f) (g) (h)

in which:

each of the symbols $R_4$ independently represents an OH group, a halogen atom, a linear or branched $C_1$-$C_6$alkyl group optionally containing a silicon atom, a linear or branched $C_1$-$C_6$alkoxy group optionally containing a silicon atom, a linear or branched $C_1$-$C_5$alkoxycarbonyl group, or a linear or branched $C_1$-$C_6$alkylsulfonamide group optionally containing a silicon atom or an amino acid function,
p represents an integer between 0 and 4, inclusive,
q represents 0 or 1.

By way of examples of UV radiation-screening particulate compounds of formula (XIV), mention may be made of the following derivatives:

2,2'-p-phenylenebis(3,1-benzoxazin-4-one), the commercial product Cyasorb® UV-3638 from the company Cytec,
2,2'-(4,4'-biphenylene)bis(3,1-benzoxazin-4-one),
2,2'-(2,6-naphthylene)bis(3,1-benzoxazin-4-one).

Another particular family of particulate organic screening agents are the polyvalent metal salts (for example $Ca^{2+}$, $Zn^{2+}$, $Mg^{2+}$, $Ba^{2+}$, $Al^{3+}$ or $Zr^{4+}$) of sulfonated or carboxylated organic screening agents, such as the polyvalent metal salts of sulfonated derivatives of benzylidenecamphor, such as those described in application FR-A 2 639 347, the polyvalent metal salts of sulfonated derivatives of benzimidazole such as those described in application EP-A-893 119, and the polyvalent metal salts of cinnamic acid derivatives, such as those described in application JP-87 166 517.

Mention may also be made of metal or ammonium or substituted ammonium complexes of UV-A and/or UV-B organic screening agents, such as those described in patent applications WO 93/10753, WO 93/11095 and WO 95/05150.

Particulate organic screening agents of triazine type or of benzotriazole type can preferably be chosen.

Among the benzotriazole insoluble screening agents, it is possible to more preferentially choose the methylenebis(hydroxyphenylbenzotriazole) derivatives having the following structure:

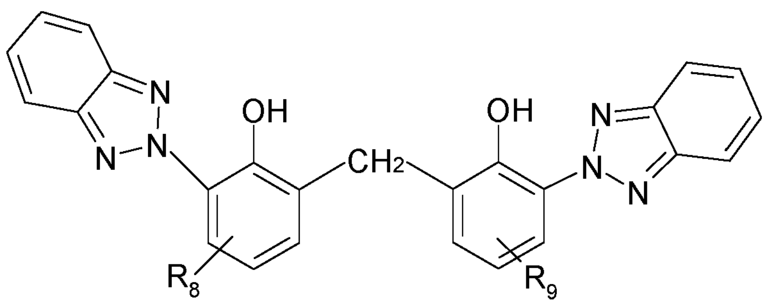

(IV)

in which $R_8$ and $R_9$, which may be identical or different, each represent a $C_1$-$C_{18}$ alkyl radical which can be substituted with one or more radicals chosen from $C_1$-$C_4$ alkyl, $C_5$-$C_{12}$ cycloalkyl, or aryl, and more particularly the compound (a) with the nomenclature 2,2'-methylenebis[6-(2H-benzotriazol-2-yl)-4-(1,1,3,3-tetramethylbutyl)phenol] or methylenebis(benzotriazolyltetramethylbutylphenol) which is sold in non-micronized form under the name Mixxim® BB/100 by the

company Fairmount Chemical and preferably in micronized form, for instance under the name Tinosorb M ® by the company Ciba Specialty Chemicals.

The particulate organic screening agents according to the invention are generally in the form of particles having a mean size ranging from 5 nm to 5 μm. More preferentially, their mean size ranges from 5 nm to 2 μm, and in particular between 5 nm and 1.5 μm, and more particularly between 10 nm and 1.0 μm.

Generally, the mean size of the particles will correspond to the mean diameter of the distribution by number.

The mean size of the particles can be determined by any conventional method, such as optical methods (quasi-elastic scattering or laser scattering), centrifuging methods or microscope visualisation and image analysis methods.

The particulate organic screening agents according to the invention can be brought to the desired particulate form by any suitable means, such as, in particular, the dry milling or milling in a solvent medium, sieving, atomisation, micronization or spraying.

The particulate organic screening agents according to the invention in a micronized form can in particular be obtained by means of a process for milling an organic UV-screening agent in the form of large-size particles in the presence of a suitable surfactant or an amphiphilic copolymer comprising at least one hydrophilic block and at least one hydrophobic block, which makes it possible to improve the dispersion of the resulting particles in cosmetic formulations.

An example of a process for the micronization of particulate organic screening agents is described in applications GB-A-2 303 549 and EP-A-893119 which are an integral part of the description. The milling apparatus used according to those documents may be an air-jet mill, a ball mill, a vibration mill or a hammer mill, and preferably a mill with high stirring speed or an impact mill and more particularly a rotary ball mill, a vibration mill, a tube mill or a rod mill.

According to this particular process, use is made, as surfactants for milling said screening agents, of alkylpolyglucosides of structure $C_nH_{2n+1}O(C_6H_{10}O_5)_xH$ in which n is an integer from 8 to 16 and x is the average degree of polymerisation of the $(C_6H_{10}O_5)$ unit and ranges from 1.4 to 1.6. They can be chosen from $C_1$-$C_{12}$ esters of a compound of structure $C_nH_{2n+1}O(C_6H_{10}O_5)_xH$ and more particularly an ester obtained by reacting a $C_1$-$C_{12}$ carboxylic acid, such as formic acid, acetic acid, propionic acid, butyric acid, sulfosuccinic acid, citric acid or tartaric acid, with one or more free OH functions on the glucoside $(C_6H_{10}O_5)$ unit.

Said surfactants are generally used at a concentration ranging from 1% to 50% by weight and more preferentially from 5% to 40% by weight relative to the weight of organic insoluble screening agent in its micronized form.

In order to improve the dispersability of the particulate organic screening agents in the cosmetic support, use may also be made of amphiphilic copolymers comprising at least one hydrophilic block and at least one hydrophobic block, such as those described in patent application EP 1 353 642.

According to a first variant, the spherical composite particles contain a core comprising at least one organic material and/or at least one inorganic material, in which are included particles of inorganic UV-screening agent.

According to this first variant, the particles of inorganic UV-screening agent are characterized by a mean elementary size generally of less than 200 nm. Advantageously, the metal oxide particles used have a mean elementary size of less than or equal to 0.15 μm.

As composite particles corresponding to this variant, mention may be made of the products Sunsil TIN 50 and Sunsil TIN 40 sold by the company Sunjin Chemical. These spherical composite particles having a mean size between 2 and 7 μm are formed of $TiO_2$ encapsulated in a silica core.

Mention may also be made of the following particles:

- spherical composite particles having a mean size of between 4 and 8 μm, containing $TiO_2$ and $SiO_2$ and having the trade name Eospoly TR, sold by the company Creations Couleurs,
- composite particles containing $TiO_2$ and a styrene/alkyl acrylate copolymer matrix, sold under the name Eospoly UV TR22 HB 50 by the company Creations Couleurs,
- composite particles containing $TiO_2$ and ZnO and a PMMA matrix and having the trade name Sun PMMA-T50, sold by the company Sunjin Chemical.

Among the composite particles A that may be used according to the invention, mention may also be made of the spherical composite particles containing $TiO_2$ and $SiO_2$, having the trade name STM ACS-0050510, supplied by the company JGC Catalysts and Chemical.

According to a second variant, the spherical composite particles A contain an inorganic UV-screening agent covered with at least one layer of an organic and/or inorganic material. According to this second variant, the particles of inorganic UV-screening agent are characterized by a mean elementary size generally of between 0.001 and 0.2 μm. Advantageously, the metal oxide particles used have a mean elementary size of between 0.01 and 0.15 μm.

According to a third variant, the spherical composite particles A contain a core made of an organic and/or inorganic material, covered with at least one layer of inorganic UV-screening agent connected to the matrix by means of a binder.

According to this third variant, the mean thickness of the layer of inorganic UV-screening agent is generally

between 0.001 and 0.2 μm and preferably between 0.01 and 0.1 μm.

The spherical composite particles A used according to the invention have a size of between 0.1 and 30 μm, preferably between 0.1 and 20 μm and even more preferentially between 0.1 and 10 μm.

According to one particular form of this variant, the spherical composite particles A can be constituted of:

i) spherical particles $A_1$ having a mean size of greater than 0.1 μm and less than 1 μm, more preferentially less than 0.6 μm and even more preferentially less than 0.4 μm, the surface of said particles $A_1$ being at least partially covered with at least one particulate solid UV-screening agent as defined previously;

ii) and optionally spherical particles $A_2$ having a mean size of greater than or equal to 2 μm, preferably greater than or equal to 3 μm, more preferentially greater than 4 μm and even better still greater than or equal to 5 μm, the surface of said particles $A_2$ being at least partially covered with at least one particulate solid UV-screening agent as defined previously.

The surface of said particles $A_1$ or $A_2$ can also be at least partially covered with at least one pulverulent colorant.

In particular, the pulverulent colorants are chosen from pigments and nacres, and mixtures thereof.

Pigments

The term "pigments" should be understood as meaning white or coloured, mineral or organic particles of any shape, which are insoluble in the physiological medium, and which are intended to colour the composition.

The pigments may be white or coloured, and mineral and/or organic.

Among the mineral pigments that may be mentioned are titanium dioxide, optionally surface-treated, zirconium oxide or cerium oxide, and also zinc oxide, iron (black, yellow or red) oxide or chromium oxide, manganese violet, ultramarine blue, chromium hydrate and ferric blue, and metal powders, for instance aluminium powder and copper powder.

The organic pigments may be chosen from the materials below, and mixtures thereof:

cochineal carmine,

organic pigments of azo dyes, anthraquinone dyes, indigoid dyes, xanthene dyes, pyrene dyes, quinoline dyes, triphenylmethane dyes or fluoran dyes.

Among the organic pigments, mention may be made especially of the D&C certified pigments known under the following names: D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5, FD&C Yellow No. 6.

The chemical materials corresponding to each of the organic colorants mentioned previously are mentioned in the publication "International Cosmetic Ingredient Dictionary and Handbook", 1997 edition, pages 371 to 386 and 524 to 528, published by "The Cosmetic, Toiletries and Fragrance Association".

A composition according to the invention may comprise a content of pigments ranging from 0 to 30% by weight relative to the total weight of the composition, preferably ranging from 0.5% to 20% by weight and preferentially ranging from 1% to 10% by weight, relative to the total weight of the composition.

Nacres

The term "nacres" should be understood as meaning coloured particles of any form, which may or may not be iridescent, especially produced by certain molluscs in their shell, or alternatively synthesized, and which have a colour effect via optical interference.

Examples of nacres that may be mentioned include nacreous pigments such as titanium mica coated with an iron oxide, mica coated with bismuth oxychloride, titanium mica coated with chromium oxide, and nacreous pigments based on bismuth oxychloride. They may also be mica particles at the surface of which are superposed at least two successive layers of metal oxides and/or of organic colorants.

The pulverulent colorant(s) can be used in the composite particles of the invention in proportions such that the ratio by weight of the particles $A_1$ or $A_2$ /colorant(s) is from 50:50 to 90:10, preferably from 50:50 to 80:20 and more preferentially from 50:50 to 70:30.

The coating of the particles $A_1$ or $A_2$ can also comprise at least one lipophilic or hydrophilic additional organic UV-screening agent.

Additional organic UV-screening agents

The additional organic screening agents are chosen in particular from dibenzoylmethane derivatives; anthranilates; cinnamic derivatives; salicylic derivatives, camphor derivatives; benzophenone derivatives; β,β-diphenyl acrylate derivatives; triazine derivatives other than those of formula (I); benzalmalonate derivatives, in particular those mentioned in patent US 5 624 663; benzimidazole derivatives; imidazolines; p-aminobenzoic acid (PABA) derivatives; benzotriazole derivatives; benzoxazole derivatives as described in patent applications EP 0 832 642, EP 1 027 883, EP 1 300 137 and DE 101 62 844; screening polymers and screening silicones such as those described in particular in application WO 93/04665; α-alkylstyrene-based dimers such as those described in patent application DE 198 55 649; 4,4-diarylbutadienes as described in applications EP 0 967 200, DE 197 46 654, DE 197 55 649, EP-A-1 008 586, EP 1 133 980 and EP 133 981; liposoluble merocyanin derivatives such as those described in applications WO 04/006 878, WO 05/058 269 and WO 06/032 741 and mixtures thereof.

As examples of additional organic UV-screening agents, mention may be made of those denoted hereinbelow under their INCI name:

Dibenzoylmethane derivatives:

Butylmethoxydibenzoylmethane, sold under the trade name Parsol 1789 by the company DSM Nutritional Products.

*para*-Aminobenzoic acid derivatives:

PABA,
Ethyl PABA,
Ethyl Dihydroxypropyl PABA,
Ethylhexyl Dimethyl PABA sold in particular under the name Escalol 507 by ISP, Glyceryl PABA,
PEG-25 PABA sold under the name Uvinul P25 by BASF.

Salicylic derivatives:

Homosalate sold under the name Eusolex HMS by Rona/EM Industries,
Ethylhexyl salicylate sold under the name Neo Heliopan OS by Haarmann and Reimer,
Dipropylene Glycol Salicylate sold under the name Dipsal by Scher,
TEA salicylate sold under the name Neo Heliopan TS by Haarmann and Reimer.

Cinnamic derivatives:

Ethylhexyl methoxycinnamate sold in particular under the trade name Parsol MCX by Hoffmann LaRoche,
Isopropyl methoxycinnamate,
Isoamyl methoxycinnamate sold under the trade name Neo Heliopan E 1000 by Haarmann and Reimer,
Cinoxate,
DEA Methoxycinnamate,
Diisopropyl Methyl Cinnamate,
Glyceryl Ethylhexanoate Dimethoxycinnamate.

β,β-Diphenylacrylate derivatives:

Octocrylene sold especially under the trade name Uvinul N539 by BASF,
Etocrylene sold in particular under the trade name Uvinul N35 by BASF.

Benzophenone derivatives:

Benzophenone-1 sold under the trade name Uvinul 400 by BASF,
Benzophenone-2 sold under the trade name Uvinul D50 by BASF,
Benzophenone-3 or oxybenzone sold under the trade name Uvinul M40 by BASF,
Benzophenone-4 sold under the trade name Uvinul MS40 by BASF, Benzophenone-5,
Benzophenone-6 sold under the trade name Helisorb 11 by Norquay,
Benzophenone-8 sold under the trade name Spectra-Sorb UV-24 by American Cyanamid,
Benzophenone-9 sold under the trade name Uvinul DS-49 by BASF, Benzophenone-12,
n-hexyl 2-(4-diethylamino-2-hydroxybenzoyl)benzoate sold under the trade name Uvinul A+ or in the form of a mixture with octyl methoxycinnamate under the trade name Uvinul A+B by BASF.

Benzylidenecamphor derivatives:

3-Benzylidene Camphor manufactured under the name Mexoryl SD by Chimex,
4-Methylbenzylidene Camphor sold under the name Eusolex 6300 by Merck,
Benzylidene Camphor Sulfonic Acid manufactured under the name Mexoryl SL by Chimex,
Camphor Benzalkonium Methosulfate manufactured under the name Mexoryl SO by Chimex,
Terephthalylidene Dicamphor Sulfonic Acid manufactured under the name Mexoryl SX by Chimex,
Polyacrylamidomethyl Benzylidene Camphor manufactured under the name Mexoryl SW by Chimex.

Phenylbenzimidazole derivatives:

Phenylbenzimidazole Sulfonic Acid sold in particular under the trade name Eusolex 232 by Merck,
Disodium phenyl dibenzimidazole tetrasulfonate sold under the trade name Neo Heliopan AP by Haarmann and Reimer.

Benzotriazole derivatives:

Drometrizole trisiloxane sold under the name Silatrizole by Rhodia Chimie.

Triazine derivatives:

Bis-Ethylhexyloxyphenol Methoxyphenyl Triazine sold under the trade name Tinosorb S by Ciba Geigy,
Ethylhexyl triazone sold in particular under the trade name Uvinul T150 by BASF, Diethylhexyl Butamido Triazone sold under the trade name Uvasorb HEB by Sigma 3V,
2,4,6-Tris(diisobutyl 4'-aminobenzalmalonate)-s-triazine,
2,4,6-Tris(dineopentyl 4'-aminobenzalmalonate)-s-triazine,
2,4-Bis(dineopentyl 4'-aminobenzalmalonate)-6-(n-butyl 4'-aminobenzoate)-s-triazine.

Anthranilic derivatives:

Menthyl anthranilate sold under the trade name Neo Heliopan MA by Haarmann and Reimer.

Imidazoline derivatives:

Ethylhexyl Dimethoxybenzylidene Dioxoimidazoline Propionate.

Benzalmalonate derivatives:

Dineopentyl 4'-methoxybenzalmalonate,
Polyorganosiloxane containing benzalmalonate functions, for instance Polysilicone-15, sold under the trade name Parsol SLX by Hoffmann LaRoche.

4,4-Diarylbutadiene derivatives:

1,1-Dicarboxy(2,2'-dimethylpropyl)-4,4-diphenylbutadiene,
and mixtures thereof.
The preferential additional organic screening agents are chosen from:

Ethylhexyl Methoxycinnamate,
Homosalate,
Ethylhexyl Salicylate,
Octocrylene,
Butylmethoxydibenzoylmethane,
Terephthalylidenedicamphorsulfonic acid,
Disodium phenyldibenzimidazoletetrasulfonate,
Phenylbenzimidazole Sulfonic Acid,
Benzophenone-3,
n-Hexyl 2-(4-diethylamino-2-hydroxybenzoyl)benzoate,
4-Methylbenzylidene Camphor,
Ethylhexyl Triazone,
Bis-Ethylhexyloxyphenol Methoxyphenyl Triazine,
Diethylhexyl Butamido Triazone,
2,4,6-Tris(dineopentyl 4'-aminobenzalmalonate)-s-triazine,
2,4,6-Tris(diisobutyl 4'-aminobenzalmalonate)-s-triazine,
2,4-Bis(dineopentyl 4'-aminobenzalmalonate)-6-(n-butyl 4'-aminobenzoate)-s-triazine,
Polysilicone-15,
Drometrizole Trisiloxane,
Dineopentyl 4'-methoxybenzalmalonate,
1,1-dicarboxy(2,2'-dimethylpropyl)-4,4-diphenylbutadiene,
and mixtures thereof.

The core of the particles $A_1$ and the core of the particles $A_2$ are constituted of an inorganic material and/or of an organic material.
The inorganic material or the organic material may be hollow or porous. The porosity of the material may be characterized by a specific surface area of from 0.05 $m^2/g$ to 1500 $m^2/g$, more preferentially from 0.1 $m^2/g$ to 1000 $m^2/g$ and more preferentially from 0.2 $m^2/g$ to 500 $m^2/g$ according to the BET method.
The inorganic materials that may be used in the core of the spherical particles $A_1$ or $A_2$ according to the present invention may be chosen from the group formed by glass, silica and aluminium oxide, and mixtures thereof.
The organic materials that may be used to form the core of the particles $A_1$ or $A_2$ are chosen from the group formed by poly(meth)acrylates, polyamides, silicones, polyurethanes, polyethylenes, polypropylenes, polystyrenes, polycaprolactams, polysaccharides, polypeptides, polyvinyl derivatives, waxes, polyesters and polyethers, and mixtures thereof.
The particles $A_1$ are partially covered with at least one layer comprising at least one particulate UV-screening agent. Preferably, at least 10% of the surface of the particle is covered, preferably at least 50% and more preferentially at least 80%, and even better still 100% of the surface of the particle is covered with solid screening agent.
The thickness of the coating of the particle $A_1$ can depend on several factors, in particular on the size of the particle. Typically, the thickness can range from 0.001 to 0.2 μm, preferably from 0.005 to 0.18 μm and more preferentially from 0.01 to 0.150 μm.
If at least two layers of coating are present on the particle $A_1$, the thickness and the composition of the layers may be identical or different.
The layer(s) of coating may comprise other materials, such as colouring pigments and/or additional UV-screening agents. These materials may be present in an amount ranging from 1% to 50% of the total weight of the materials

coating the particle $A_1$.

The spherical particles $A_2$ have a mean size of greater than or equal to 2 μm, preferably greater than or equal to 3 μm and more preferentially greater than 4 μm. The mean size can be limited to at most 50 μm, preferably at most 30 μm, more preferentially at most 20 μm and even more particularly at most 10 μm.

The mean particle size or the mean diameter is an arithmetic mean diameter and can be determined, for example, by calculating the mean of the dimensions of about 100 particles chosen on an image obtained by scanning electron microscopy.

The spherical particles $A_2$ may be hollow or solid and preferably solid.

According to one particularly preferred form, the core of the particles $A_1$ will be constituted of styrene copolymer and more preferentially of styrene /acrylate copolymer and more particularly of styrene/methylacrylate copolymer, for instance the product sold under the trade name Sunspheres by the company Rohm & Haas and also the product sold under the trade names SX859(A) and SX866(B) by the company JSR Corp in Japan.

According to one particularly preferred form, the core of the particles $A_2$ will be constituted of

- polymethacrylate and more particularly of poly(methyl methacrylate), for instance the product sold under the trade name MR-7GC by Soken in Japan,
- polyamide, for instance the product sold under the trade name SP-500 by Toray or Orgasol by Arkema,
- polytetrafluoroethylene, such as the commercial product sold under the name Ceridust 9205F by Clariant.

The weight ratio of the particles $A_1$/particles $A_2$ can be from 10:90 to 90:10, preferably from 20:80 to 80:20 and more preferentially from 30:70 to 70:30.

The weight ratio of the particles $A_1$ to the particulate UV-screening agent can be from 10:90 to 90:10, preferably from 20:80 to 80:20 and more preferentially from 30:70 to 70:30.

The weight ratio of the particles $A_1$/particles $A_2$/particulate UV-screening agent can be from 20:50:30 to 50:20:30, preferably from 35:15:50 to 15:35:50, more preferentially from 10:20:70 to 20:10:70 and more particularly 50:20:30 or 35:15:50.

According to one particularly preferred form, the spherical composite particles A comprise:

i) spherical particles $A_1$ having a mean size of greater than 0.1 μm and less than 1 μm, more preferentially less than 0.6 μm and even more preferentially less than 0.4 μm, the surface of said particles $A_1$ being at least partially covered with titanium dioxide particles and the core of the particles being constituted of styrene/methyl methacrylate copolymer;

ii) spherical particles $A_2$ having a mean size of greater than or equal to 2 μm, preferably greater than or equal to 3 μm, more preferentially greater than 4 μm and even better still greater than or equal to 5 μm, the surface of said particles $A_2$ being covered with at least titanium dioxide particles and the core of the particles being constituted of poly(methyl methacrylate).

According to another particularly preferred form, the spherical hollow composite particles A comprise:

i) spherical particles $A_1$ having a mean size of greater than 0.1 μm and less than 1 μm, more preferentially less than 0.6 μm and even more preferentially less than 0.4 μm, the surface of said particles $A_1$ being at least partially covered with particles of particulate organic UV-screening agent and the core of the particles being constituted of styrene/methyl methacrylate copolymer;

ii) spherical particles $A_2$ having a mean size of greater than or equal to 2 μm, preferably greater than or equal to 3 μm, more preferentially greater than 4 μm and even better still greater than or equal to 5 μm, the surface of said particles $A_2$ being covered with at least particles of particulate organic UV-screening agent and the core of the particles being constituted of poly(methyl methacrylate).

Even more particularly, the particulate organic UV-screening agent will be the compound 2,2'-methylenebis[6-(2H-benzotriazol-2-yl)-4-(1,1,3,3-tetramethylbutyl)phenol] or methylenebisbenzotriazolyltetramethylbutylphenol sold in solid form under the name Mixxim® BB/100 by the company Fairmount Chemical and in micronized form under the name Tinosorb M ® by the company Ciba Specialty Chemicals.

<u>Mechanofusion process</u>

The composite particles A according to the invention can be prepared by subjecting the following to a mechanofusion process:

- the spherical particles $A_1$ having a mean size of greater than 0.1 μm and less than 1 μm, more preferentially less than 0.6 μm and even more preferentially less than 0.4 μm,
- and optionally the spherical particles $A_2$ having a mean size of greater than or equal to 2 μm, preferably greater than or equal to 3 μm, more preferentially greater than 4 μm and even better still greater than or equal to 5 μm, and
- at least one solid UV-screening agent as previously defined,
- and optionally the additional UV-screening agent(s) and/or the pulverulent colorant(s) as previously defined.

A mechanofusion process consists of a process in which a mechanical power, such as a compressive force, a frictional force or a shear force, is exerted on a plurality of elements, causing the fusion of said elements.

The mechanofusion process can be performed with a device comprising a rotary chamber and an internal part attached to a scraper, such as the device sold under the trade name Hosokawa Micron Corporation in Japan.

A process of hybridization by mechanofusion will preferably be used.

The hybridization process was developed in the 1980s. It is a type of mechanofusion process in which a strong mechanical power is applied to a plurality of particles in order to cause a mechanochemical reaction so as to form composite particles.

According to the hybridization process, the mechanical power is produced by a high-speed rotor which can have a diameter ranging from 10 cm to 1 m and which can rotate at a speed ranging from 1000 to 10 000 revolutions/minute. The hybridization process can be carried out in air or under a dry atmosphere. Indeed, the high-speed rotation of the rotor can generate a high-speed air flow in proximity to the rotor. Liquid materials can be subjected to the hybridization process in the presence of solid materials.

The hybridization process can be carried out using a hybridization system sold under the trade name Nara Machinery, in which at least two types of particles, generally particles comprising a core and fine particles, are introduced into a hybridizer equipped with a high-speed rotor having a plurality of blades in a dry chamber, and the particles are dispersed in the chamber and mechanical and thermal energy is produced on the particles (compression, friction and shear) for a short period such as from 1 to 10 minutes and preferably from 1 to 5 minutes. This results in particles of one type (fine particles) integrated on or attached to particles of another type (i.e. particles comprising a core) so as to form composite particles. It is preferable for the particles to be subjected to an electrostatic treatment, for example by shaking them so as to form an "ordered mixture" in which particles of one type are spread out so as to cover the particles of the other type. The hybridization process can be carried out using a Theta composer sold by Tokuju Corporation.

The hybridization process can be carried out using a device of the Composi Hybrid or Mechano Hybrid type sold by Nippon Coke.

According to the invention, the hollow particles $A_1$, the particles $A_2$, the particulate screening agent(s), the optional colouring pigment(s) and the optional additional screening agent(s) are introduced into a hybridizer so as to form a composite pigment.

The hybridization process can be carried out using a rotor rotating at approximately 8000 rpm for approximately 3 minutes.

## PARTICLES B OF INORGANIC UV-SCREENING AGENT

The inorganic UV-screening agent constituting these particles B is chosen from metal oxides, and in particular titanium oxides or mixtures thereof. Particularly preferably, the inorganic UV-screening agent is titanium dioxide ($TiO_2$).

In particular, the titanium dioxide ($TiO_2$) can be in the rutile and/or anatase form and/or in an amorphous form.

The particles B of inorganic UV-screening agent, in particular of metal oxide, preferably have a mean elementary size generally of between 0.07 μm and 0.2 μm.

According to one particular form of the invention, the particles B can be in powder form.

The particles B of inorganic UV-screening agent are treated with at least one surface-treatment agent.

The particles B of inorganic UV-screening agent, which are hydrophobically modified, may be coated by undergoing one or more surface treatments of chemical, electronic, mechanochemical and/or mechanical nature with compounds as described, for example, in Cosmetics & Toiletries, February 1990, Vol. 105, pp. 53-64, such as amino acids, beeswax, fatty acids, fatty alcohols, anionic surfactants, lecithins, sodium, potassium, zinc, iron or aluminium salts of fatty acids, metal alkoxides (titanium or aluminium alkoxides), polyethylene, silicones, proteins (collagen, elastin), alkanolamines, silicon oxides, metal oxides or sodium hexametaphosphate.

As is known, silicones are organosilicon polymers or oligomers of linear or cyclic, branched or crosslinked structure, of variable molecular weight, obtained by polymerization and/or polycondensation of suitably functionalized silanes, and are essentially constituted of a repetition of main units in which the silicon atoms are linked together via oxygen atoms (siloxane bond), optionally substituted hydrocarbon-based radicals being directly attached to said silicon atoms via a carbon atom. The term "silicones" also includes the silanes required for their preparation, in particular alkylsilanes.

Preferably, use will be made of metal oxide particles coated with at least one surface-treatment agent chosen from a dimethicone and a linear or branched $C_{12}$-$C_{18}$ fatty acid and more particularly stearic acid.

The hydrophobically-modified particles B of inorganic UV-screening agent, in particular of metal oxide, may also be treated with other surface-treatment agents, in particular with cerium oxide, alumina, silica, aluminium compounds such as aluminium hydroxide, or silicon compounds, or mixtures thereof.

The particles B of inorganic UV-screening agent are metal oxides treated with at least one surface-treatment agent, in hydrophobically-modified coated form, chosen from :

- a dimethicone-treated titanium dioxide;
- a titanium dioxide treated with a silica/alumina/stearic acid or alumina/stearic acid mixture;
- an oily dispersion of $TiO_2$ particles treated with a mixture of stearic acid, polyhydroxystearic acid and alumina.

By way of non-limiting example of hydrophobically-treated titanium dioxide particles, mention may be made of dimethicone-treated titanium dioxide, such as the product sold by the company Tayca under the trade name MTY-700BS; a titanium dioxide treated with a silica/alumina/stearic acid or alumina/stearic acid mixture.

According to one particular form of the invention, the particles B can be in the form of an oily dispersion. The oil present in the oily dispersion of particles of inorganic UV-screening agent is preferably chosen from $C_{12}$-$C_{15}$ alkyl benzoates or triglycerides such as caprylic/capric triglyceride.

The oily dispersions of hydrophobically-modified particles B of inorganic UV-screening agent may also comprise at least one dispersant, for instance polyhydroxystearic acid.

Use will be made more particularly of the following oily dispersions of $TiO_2$ particles treated with a mixture of stearic acid, polyhydroxystearic acid and alumina:

- titanium dioxide (and) $C_{12}$-$C_{15}$ alkyl benzoate (and) polyhydroxystearic acid (and) stearic acid (and) alumina, for instance the product sold under the trade name Solaveil XT-100 by the company Croda;
- titanium dioxide (and) caprylic/capric triglyceride (and) polyhydroxystearic acid (and) stearic acid (and) alumina, for instance the product sold under the trade name Solaveil XT-300 by the company Croda.

Preferably, the content of particles B of inorganic UV-screening agent in the composition according to the invention is between 0.1% and 40%, preferably between 0.5% and 30% by weight and even more preferentially between 1% and 20% relative to the total weight of the composition.

## AQUEOUS PHASE

The aqueous phase of the compositions of the invention contains water and optionally other water-soluble or water-miscible organic solvents.

An aqueous phase suitable for the invention can comprise, for example, a water chosen from a natural spring water, such as water from La Roche-Posay, water from Vittel or waters from Vichy, or a floral water.

The water-soluble or water-miscible solvents suitable for the invention comprise short-chain monoalcohols, for example $C_1$-$C_4$ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.

According to a preferred embodiment, use may more particularly be made of ethanol, propylene glycol, glycerol, and mixtures thereof.

The water or the aqueous phase may be present in a composition of the invention in a content ranging from 5% to 90% by weight, in particular from 5% to 75% by weight and more particularly from 10% to 70% by weight, relative to the total weight of said composition.

A water-soluble organic solvent may be present in a composition of the invention in a content ranging from 1% to 30% by weight and in particular from 2% to 20% by weight, relative to the total weight of said composition.

## OILY PHASE

The composition of the invention may also comprise at least one oily phase comprising at least one oil.

For the purposes of the invention, the term "oily phase" is understood to mean a phase comprising at least one oil and all of the liposoluble and lipophilic ingredients and the fatty substances used for the formulation of the compositions of the invention.

The term "oil" is understood to mean any fatty substance in the liquid form at ambient temperature (20 - 25°C) and at atmospheric pressure (760 mmHg).

An oil suitable for the invention can be volatile or non-volatile.

An oil suitable for the invention can be chosen from hydrocarbon-based oils, silicone oils, fluorinated oils and mixtures thereof.

A hydrocarbon-based oil suitable for the invention can be an animal hydrocarbon-based oil, a vegetable hydrocarbon-based oil, a mineral hydrocarbon-based oil or a synthetic hydrocarbon-based oil.

An oil suitable for the invention can advantageously be chosen from mineral hydrocarbon-based oils, vegetable hydrocarbon-based oils, synthetic hydrocarbon-based oils, silicone oils and mixtures thereof.

For the purposes of the present invention, the term "silicone oil" is understood to mean an oil comprising at least one silicon atom, and in particular at least one Si-O group.

The term "hydrocarbon-based oil" is understood to mean an oil comprising mainly hydrogen and carbon atoms.

The term "fluorinated oil" is understood to mean an oil comprising at least one fluorine atom.

A hydrocarbon-based oil suitable for the invention can in addition optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent or agents, at least one volatile or non-volatile hydrocarbon-based oil and/or one volatile and/or non-volatile silicone oil.

For the purposes of the invention, the term "volatile oil" is understood to mean an oil capable of evaporating on contact with the skin or the keratin fibre in less than one hour, at ambient temperature and atmospheric pressure. The volatile oil(s) of the invention are volatile cosmetic oils which are liquid at ambient temperature and which have a non-zero vapour pressure, at ambient temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at ambient temperature and atmospheric pressure for at least several hours, and that especially has a vapour pressure of less than $10^{-3}$ mmHg (0.13 Pa).

Hydrocarbon-based oils

Mention may in particular be made, as non-volatile hydrocarbon-based oils which can be used according to the invention, of:

(i) hydrocarbon-based oils of vegetable origin such as triglyceride esters, which are generally fatty acid triesters of glycerol, the fatty acids of which may have chain lengths varying from $C_4$ to $C_{24}$, these chains possibly being linear or branched, and saturated or unsaturated; these oils are especially wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion-flower oil and musk rose oil; or else caprylic/capric acid triglycerides, for instance those sold by the company Stearineries Dubois or those sold under the names Miglyol 810, 812 and 818 by the company Dynamit Nobel;

(ii) synthetic ethers containing from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, such as the oils of formula RCOOR' in which R represents the residue of a linear or branched fatty acid comprising from 1 to 40 carbon atoms and R' represents a hydrocarbon-based chain, in particular a branched hydrocarbon-based chain, comprising from 1 to 40 carbon atoms, provided that R + R' is ≥10, such as, for example, purcellin oil (cetearyl octanoate), isopropyl myristate, isopropyl palmitate, $C_{12}$-$C_{15}$ alkyl benzoate, such as the product sold under the trade name Finsolv TN or Witconol TN by Witco or Tegosoft TN by Evonik Goldschmidt, 2-ethylphenyl benzoate, such as the commercial product sold under the name X-Tend 226 by ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI by Enichem Augusta Industriale, and also di(linear $C_{14}$-$C_{15}$ alkyl) tartrates, such as those sold under the name Cosmacol ETL by the same company; or acetates;

(v) fatty alcohols which are liquid at ambient temperature, comprising a branched and/or unsaturated carbon chain having from 12 to 26 carbon atoms, such as octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates such as dicaprylyl carbonate, for instance the product sold under the name Cetiol CC by the company Cognis;

(viii) fatty amides, such as isopropyl N-lauroyl sarcosinate, such as the product sold under the trade name Eldew SL205 from Ajinomoto;
and mixtures thereof.

Mention may in particular be made, as volatile hydrocarbon-based oils which can be used according to the invention, of hydrocarbon-based oils having from 8 to 16 carbon atoms, in particular branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, or the alkanes described in the patent applications from Cognis, WO 2007/068371 or WO 2008/155059 (mixtures of different alkanes differing by at least one carbon). These alkanes are obtained from fatty alcohols, which are themselves obtained from coconut oil or palm oil, the oils sold under the trade name Isopar or Permethyl, branched $C_8$-$C_{16}$ esters, isohexyl neopentanoate, and mixtures thereof.
Other volatile hydrocarbon-based oils, for instance petroleum distillates, especially those sold under the name Shell Solt by the company Shell, may also be used. According to one embodiment, the volatile solvent is chosen from volatile hydrocarbon-based oils having from 8 to 16 carbon atoms, and mixtures thereof.

<u>Silicone oils</u>

The non-volatile silicone oils can be chosen in particular from non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.
Mention may be made, as volatile silicone oils, for example, of volatile linear or cyclic silicone oils, in particular those having a viscosity ≤ 8 centistokes ($8\times10^{-6}$ $m^2/s$) and having in particular from 2 to 7 silicon atoms, these silicones optionally comprising alkyl or alkoxy groups having from 1 to 10 carbon atoms. Mention may in particular be made, as volatile silicone oil which can be used in the invention, of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane and mixtures thereof.
Mention may also be made of the volatile linear alkyltrisiloxane oils of general formula (I):

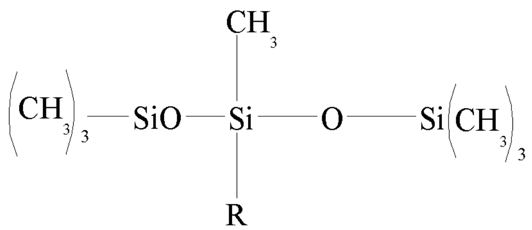

where R represents an alkyl group comprising from 2 to 4 carbon atoms, one or more hydrogen atoms of which can be replaced by a fluorine or chlorine atom.
Mention may be made, among the oils of general formula (I), of:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

Fluorinated oils

Use may also be made of volatile fluorinated oils, such as nonafluoromethoxybutane, decafluoropentane, tetradecafluorohexane, dodecafluoropentane, and mixtures thereof.
The oily phase (excluding lipophilic organic UV-screening agent) preferably ranges from 3% to 60% by weight and preferably from 5% to 30% by weight relative to the total weight of the composition.
An oily phase according to the invention can additionally comprise other fatty substances, mixed with or dissolved in the oil.
Another fatty substance which can be present in the oily phase can be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

**GALENICAL FORMS**

The compositions according to the invention can be prepared according to the techniques well known to those skilled in the art.
They may be in the form of an aqueous lotion or of an aqueous gel or comprise, in addition to the aqueous phase, at least one fatty phase and may be in the form of a simple or complex emulsion (O/W, W/O, O/W/O or W/O/W) such as a milk, of a cream or of a cream gel. They may optionally be packaged as an aerosol and may be in the form of a spray.
Preferably, the compositions according to the invention are in the form of an oil-in-water or water-in-oil emulsion, and more preferentially in water-in-oil form.
The emulsification processes that may be used are of paddle or impeller, rotor-stator and high-pressure homogenizer (HPH) type.
To obtain stable emulsions with a low content of emulsifying compounds (oil/emulsifier ratio > 25), it is possible to make the dispersion in concentrated phase and then to dilute the dispersion with the rest of the aqueous phase.
It is also possible, via HPH (between 50 and 800 bar), to obtain stable dispersions with drop sizes which can fall to 100 nm.
The emulsions generally comprise at least one emulsifier chosen from amphoteric, anionic, cationic or non-ionic emulsifiers, used alone or as a mixture. The emulsifiers are appropriately chosen according to the emulsion to be obtained (W/O or O/W). The emulsions may also contain stabilizers of other types, for instance fillers, or gelling or thickening polymers.
For the O/W emulsions, examples of emulsifiers that may be mentioned include non-ionic emulsifiers such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG-100 stearate/glyceryl stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by the company Henkel under the respective names Plantaren 2000 and Plantaren 1200, cetostearyl glucoside, optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of the mixture of arachidyl alcohol, behenyl alcohol and arachidyl glucoside, sold under the name Montanov 202 by the company SEPPIC. According to one particular embodiment of the invention, the mixture of the alkylpolyglucoside as defined above with the corresponding fatty alcohol can be in the form of a self-emulsifying composition, for example as described in document WO-A-92/06778.
Among the other emulsion stabilizers, use may also be made of isophthalic acid or sulfoisophthalic acid polymers, and in particular phthalate/sulfoisophthalate/glycol copolymers, for example the diethylene glycol/phthalate/isophthalate/1,4-cyclohexanedimethanol copolymer (INCI name: Polyester-5) sold under the names Eastman AQ Polymer (AQ35S, AQ38S, AQ55S and AQ48 Ultra) by the company Eastman Chemical.
Among the other emulsion stabilizers, mention may also be made of hydrophobically modified 2-acrylamido-2-methylpropanesulfonic acid polymers such as those described in patent application EP 1 069 142.

When it is an emulsion, the aqueous phase of the latter can comprise a non-ionic vesicular dispersion prepared according to known processes (Bangham, Standish and Watkins, J. Mol. Biol., 13, 238 (1965), FR 2 315 991 and FR 2 416 008).

As emulsifying surfactants that can be used for the preparation of W/O emulsions, mention may be made, for example, of emulsifying surfactants having an HLB of less than or equal to 5 at 25°C.

The term HLB (hydrophilic lipophilic balance) is well known to those skilled in the art, and denotes the hydrophilic-lipophilic balance of a surfactant.

The HLB of the surfactant(s) used according to the invention is the HLB according to Griffin, defined in the publication J. Soc. Cosm. Chem. 1954 (volume 5), pages 249-256.

Non-limiting examples of surfactants with an HLB of less than or equal to 5 are especially given in the publication entitled McCutcheon's Emulsifiers & Detergents, 1998 International Edition, MC Publishing Company, in the chapter entitled HLB Index.

Examples of W/O emulsifying surfactants that may be mentioned include alkyl esters or ethers of sorbitan, of glycerol, of polyol or of sugars; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R by the company Goldschmidt, and the mixture of cetyldimethicone copolyol, of polyglyceryl isostearate (4 mol) and of hexyl laurate, sold under the name Abil WE O9 by the company Goldschmidt. One or more coemulsifiers may also be added thereto, which may be chosen advantageously from the group consisting of polyol alkyl esters.

Non-silicone emulsifiers, in particular alkyl esters or ethers of sorbitan, of glycerol, of polyol or of sugars, will be preferred.

Alkyl esters of polyol that may in particular be mentioned include polyethylene glycol esters, for instance PEG-30 dipolyhydroxystearate, such as the product sold under the name Arlacel P135 by the company ICI.

Esters of glycerol and/or of sorbitan that may be mentioned include, for example, polyglyceryl isostearate, such as the product sold under the name Isolan GI 34 by the company Goldschmidt, sorbitan isostearate, such as the product sold under the name Arlacel 987 by the company ICI, sorbitan glyceryl isostearate, such as the product sold under the name Arlacel 986 by the company ICI, and mixtures thereof.

According to one particularly preferred form of the invention, the compositions are in the form of a water-in-oil emulsion.

## ADDITIVES

The compositions in accordance with the present invention may also comprise standard cosmetic adjuvants chosen in particular from organic solvents, ionic or non-ionic, hydrophilic or lipophilic thickeners, demulcents, humectants, opacifiers, stabilizers, emollients, silicones, antifoams, fragrances, preserving agents, anionic, cationic, non-ionic, zwitterionic or amphoteric surfactants, fillers, polymers, propellants, basifying or acidifying agents or any other ingredient commonly used in the cosmetics and/or dermatological field.

Among the organic solvents that may be mentioned are lower alcohols and polyols. The latter can be chosen from glycols and glycol ethers, such as ethylene glycol, propylene glycol, butylene glycol, dipropylene glycol or diethylene glycol.

Hydrophilic thickeners that may be mentioned include carboxyvinyl polymers such as the Carbopol products (carbomers) and the Pemulen products (acrylate/$C_{10}$-$C_{30}$-alkyl acrylate copolymer); polyacrylamides, for instance the crosslinked copolymers sold under the names Sepigel 305 (CTFA name: polyacrylamide/$C_{13}$-$C_{14}$ isoparaffin/Laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Clariant under the trade name Hostacerin AMPS (CTFA name: ammonium polyacryloyldimethyl taurate) or Simulgel 800 sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, for instance Simulgel NS and Sepinov EMT 10 sold by the company SEPPIC; cellulose derivatives such as hydroxyethyl cellulose; polysaccharides and especially gums such as xanthan gum; and mixtures thereof.

Lipophilic thickeners that may be mentioned include synthetic polymers such as poly($C_{10}$-$C_{30}$ alkyl acrylates) sold under the name Intelimer IPA 13-1 and Intelimer IPA 13-6 by the company Landec, or modified clays such as hectorite and its derivatives, for instance the products sold under the name Bentone.

The compositions according to the invention may also furthermore comprise additional cosmetic and dermatological active agents.

Mention may be made, among active agents, of:

- vitamins (A, C, E, K, PP, and the like) and their derivatives or precursors, alone or as mixtures;
- antioxidants;
- free-radical scavengers;
- antiglycation agents;
- soothing agents;
- NO-synthase inhibitors;
- agents which stimulate the synthesis of dermal or epidermal macromolecules and/or which prevent their decomposition;
- agents which stimulate the proliferation of fibroblasts;
- agents which stimulate the proliferation of keratinocytes;
- muscle relaxants;
- tensioning agents;
- mattifying agents;
- keratolytic agents;
- desquamating agents;
- moisturizers, for instance polyols such as glycerol, butylene glycol or propylene glycol;
- anti-inflammatory agents;
- agents which act on the energy metabolism of cells;
- insect repellents;
- substance P antagonists or CGRP antagonists;
- agents for combating hair loss and/or for restoring the hair;
- anti-wrinkle agents;
- additional inorganic and organic UV-screening agents such as those previously mentioned.

Needless to say, those skilled in the art will take care to select the optional additional compound(s) mentioned above and/or the amounts thereof such that the advantageous properties intrinsically associated with the compositions in accordance with the invention are not, or are not substantially, adversely affected by the envisaged addition(s).
Those skilled in the art will choose said active agent(s) according to the desired effect on the skin, hair, eyelashes, eyebrows or nails.
The cosmetic compositions according to the invention have applications in a great number of treatments, in particular cosmetic treatments, of the skin, lips and hair, including the scalp.
Another subject of the present invention is constituted of the use of the compositions according to the invention as defined above in the manufacture of products for the cosmetic treatment of the skin, lips, nails, hair, eyelashes, eyebrows and/or scalp, in particular care products, anti-sun products and make-up products.
The cosmetic compositions according to the invention can be used, for example, as make-up products.
The cosmetic compositions according to the invention may be used, for example, as care products and/or anti-sun protection products for the face and/or the body, of liquid to semi-liquid consistency, such as milks, more or less rich creams, cream-gels and pastes. They may optionally be packaged in aerosol form and may be in the form of a mousse or a spray.
The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.
The compositions packaged as an aerosol in accordance with the invention generally comprise conventional propellants, such as, for example, hydrofluorinated compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.
The examples that follow serve to illustrate the invention.

## EXAMPLES

**Examples 1 to 4:** Preparation of composite pigments by means of a mechanofusion hybridization process

The constituents indicated in Table 1 were subjected to hybridization using a hybridizer equipped with a high-speed rotor having a plurality of blades in a chamber under dry conditions, sold by Nara Machinery Co., Ltd in Japan,

making it possible to obtain the composite pigments of Examples 1 and 2.

For each Example 1 and 2, the constituents indicated in Table 1 were mixed, in the weight ratio indicated in the same table, in a plastic bag which is shaken for a few minutes. The mixture is then placed in the hybridizer and the rotor rotates at 8000 rpm (linear speed of 100 m/s) for 3 minutes so as to obtain the composite pigments of Examples 1 and 2.

| Composite pigments A | Particles $A_1$ | Particles $A_2$ | Particulate UV-screening agent | |
|---|---|---|---|---|
| | Styrene/Acrylate Copolymer(1) | PMMA (2) | $TiO_2$(3) | Methylenebisbenzotriazolyl tetramethylbutylphenol (4) |
| Particle size | 350 nm | 6 μm | 15 nm | |
| Ex. 1 | 35 | 15 | 50 | |
| Ex. 2 | 50 | 20 | 30 | |
| Ex. 3 | 35 | 15 | | 50 |
| Ex. 4 | 50 | 20 | | 30 |

(1) Styrene/Acrylate Copolymer: Sunspheres sold by Rohm and Haas
(2) PMMA: MR-7GC sold by Soken
(3) $TiO_2$: MT-100 TV sold by Tayca
(4) Tinosorb-M: Methylenebisbenzotriazolyltetramethylbutylphenol sold by BASF

**Examples 5 to 7: anti-sun water/oil emulsions**

Compositions 5 to 7 below were prepared. The ingredients are given as weight percentages of active material relative to the total weight of the composition.

| Phase | Ingredients | Ex5 | Ex6 | Ex 7 (invention) | Ex8 (invention) |
|---|---|---|---|---|---|
| **$A_1$** | PEG-30 dipolyhydroxystearate (Arlacel P135) | 2 | 2 | 2 | 2 |
| | Dicaprylyl carbonate (Cetiol CC) | 10 | 10 | 10 | 10 |
| | Caprylic/capric acid triglycerides (60/40) | 5 | 5 | 5 | 5 |
| | 1,2-Octanediol | 0.3 | 0.3 | 0.3 | 0.3 |
| **$A_2$** | Dimethicone | 2 | 2 | 2 | 2 |
| **$A_3$** | Oxyethylenated and oxypropylenated (35/3) (18 EO/ 18 PO) poly methyllauryl/methylsiloxane (Dow Corning 200) | 2 | 2 | 2 | 2 |
| | Vitamin E | 0.1 | 0.1 | 0.1 | 0.1 |
| **$B_1$** | Water | 18 | 18 | 18 | 18 |
| | EDTA | 0.1 | 0.1 | 0.1 | 0.1 |
| | Sodium chloride | 1 | 1 | 1 | 1 |
| | Magnesium sulfate | 0.5 | 0.5 | 0.5 | 0.5 |
| **$B_2$** | Glycerol | 4 | 4 | 4 | 4 |
| | Glycol | 4 | 4 | 4 | 4 |
| **C** | Isohexadecane | 12.21 | 9.82 | 11.02 | 13.11 |
| **D** | Titanium dioxide (and) C12-C15 Alkyl benzoate (and) Polyhydroxystearic acid (and) Stearic acid (and) Alumina (Solaveil XT-100) **(47% by weight of $TiO_2$)** | 29.79 | - | 14.89 | 14.89 |

(continued)

| Phase | Ingredients | Ex5 | Ex6 | Ex 7 (invention) | Ex8 (invention) |
|---|---|---|---|---|---|
| D | Composite particles according to Example 1 **(50% by weight of $TiO_2$)** | - | - | - | 14.00 |
| | Spherical composite particles having a mean size of between 2 and 7 µm are formed from $TiO_2$ encapsulated in a silica matrix (Sunsil Tin 50 - **43.5% by weight of $TiO_2$)** | - | 32.18 | 16.09 | - |
| | Iron oxides | 2 | 2 | 2 | 2 |
| E | Alcohol | 7 | 7 | 7 | 7 |

Emulsion preparation method:

The oily phase $A_1$ is prepared by mixing the starting materials with mechanical stirring at 80-85°C. The phases $A_2$ and $A_3$ are added after cooling of $A_1$ to 60-65°C. The aqueous phase ($B_1$ and $B_2$) is prepared by mixing the starting materials with mechanical stirring at ambient temperature. The solutions obtained are macroscopically homogeneous. The emulsion is prepared by slow introduction of the aqueous phase into the oily phase with stirring using a homogenizer of Moritz type at a stirring speed of 4500 revolutions per minute for 10 minutes. The oily phase C is added to the emulsion obtained with gentle stirring, and then phases D and E are added. The emulsion obtained is cooled to ambient temperature, with slow stirring. It is characterized by drops having a size of between 1 µm and 10 µm.

These compositions were evaluated according to the following properties:

- screening efficacy, and
- sensoriality on application to the skin.

Examples 5 to 7 were compared with an equal content of $TiO_2$ at 14%:

***In vitro* protocol for evaluating the screening efficacy**

*In vitro* SPF

The sun protection factor (SPF) is determined according to the *in vitro* method described by B. L. Diffey in J. Soc. Cosmet. Chem. 40, 127-133 (1989). The measurements were carried out using a UV-1000S spectrophotometer from the company Labsphere. Each composition is applied to a rough plate of PMMA in the form of a uniform and even deposit in a proportion of 1 $mg/cm^2$.

*In vitro* PPD index

To measure the UV-A protection index, the PPD (persistent pigment darkening) method, which measures the skin colour observed 2 to 4 hours after exposing the skin to UV-A, is particularly recommended and used. This method has been adopted since 1996 by the Japanese Cosmetic Industry Association (JCIA) as the official test procedure for the UV-A labelling of products and is frequently used by test laboratories in Europe and the United States (Japan Cosmetic Industry Association Technical Bulletin. Measurement Standards for UVA protection efficacy. Issued November 21, 1995 and effective as of January 1, 1996).

The $UVA_{PPD}$ sun protection factor ($UVA_{PPD}$ PF) is expressed mathematically by the ratio of the UV-A radiation dose necessary to reach the pigmentation threshold with the UV-screening agent (MPPDp) to the UV-A radiation dose necessary to reach the pigmentation threshold without UV-screening agent (MPPDnp).

$$UVA_{PPD}PF = \frac{MPPDp}{MPPDnp}$$

The measurements were carried out according to an *in vitro* test using a UV-2000S spectrophotometer from the company Labsphere. Each composition is applied to a rough plate of PMMA in the form of a uniform and even deposit

in a proportion of 1 mg/cm$^2$.

Protocol for evaluating the sensory effect after application to the skin

The sensory effect after application of the formula to the skin is evaluated by applying the formula to a forearm at a rate of 2 mg/cm$^2$, waiting for a drying time equal to 2 minutes and then assessing the friction force felt between the fingers and the surface of the forearm.

Results

| **Examples** | ***in vitro* SPF** | ***in vitro* PPD** | **Sensoriality** | |
|---|---|---|---|---|
| | | | Greasy | Crunchy |
| **Example 5** | 23.8 +/- 3.1 | 12.2 +/- 1.2 | +++ | - |
| **Example 6** | 5.7 +/- 0.8 | 2.9 +/- 0.3 | -- | +++ |
| **Example 7 (invention)** | 71.2 +/- 8.9 | 15.9 +/- 0.9 | - | - |

These results show that the combination, in an aqueous medium, of the composite particles A and the composite particles B according to the invention makes it possible to obtain a photoprotective composition which exhibits considerable efficacy and good cosmeticity, contrary to the compositions containing each of the types of screening particles individually at the same $TiO_2$ content.

**Claims**

**1.** Composition containing, in a cosmetically acceptable medium, at least:

a) at least one aqueous phase and
b) composite particles A in spherical form, having a mean size of between 0.1 and 30 μm comprising at least one particulate UV-screening agent and a core constituted of at least one inorganic material and/or of at least one organic material, and
c) free particles B of inorganic UV-screening agent having a mean elementary size greater than 0.07 μm,

the content of composite particles A in the composition according to the invention ranges from 1% to 70% relative to the total weight of the cosmetic composition. the content of particles B in the composition according to the invention ranges from 0.1% and 40% relative to the total weight of the composition,
the core of the spherical composite particles A contains a material or a mixture of materials chosen from:

- $SiO_2$,
- poly(methyl methacrylate),
- copolymers of styrene and of a C1/C5 alkyl (meth)acrylate derivative,

the particulate UV-screening agent is inorganic and chosen from titanium oxides,
the particles B of inorganic UV-screening agent are metal oxides treated with at least one surface-treatment agent, in hydrophobically-modified coated form, chosen from :

- a dimethicone-treated titanium dioxide;
- a titanium dioxide treated with a silica/alumina/stearic acid or alumina/stearic acid mixture;
- an oily dispersion of $TiO_2$ particles treated with a mixture of stearic acid, polyhydroxystearic acid and alumina.

**2.** Composition according to Claim 1, in which the composite particles A contain a core comprising at least one organic material and/or at least one inorganic material, in which are included particles of particulate UV-screening agent.

**3.** Composition according to Claim 1, in which the spherical composite particles A contain a core made of an organic

material and/or an inorganic material, covered with at least one layer of particulate UV-screening agent which may be connected to the matrix by means of a binder.

**4.** Composition according to Claim 1, in which the composite particles A contain a particulate UV-screening agent covered with at least one layer of an organic material and/or of an inorganic material.

**5.** Composition according to any one of Claims 1 to 4, in which the content of composite particles A in the composition according to the invention ranges from 1.5% to 50% and preferably from 2% to 40% by weight relative to the total weight of the cosmetic composition.

**6.** Composition according to any one of Claims 1 to 5, in which the spherical composite particles A can be constituted of:

i) spherical particles $A_1$ having a mean size of greater than 0.1 μm and less than 1 μm, more preferentially less than 0.6 μm and even more preferentially less than 0.4 μm, the surface of said particles $A_1$ being at least partially covered with at least one particulate solid UV-screening agent as defined in the preceding claims;
ii) and optionally spherical particles $A_2$ having a mean size of greater than or equal to 2 μm, preferably greater than or equal to 3 μm, more preferentially greater than 4 μm and even better still greater than or equal to 5 μm, the surface of said particles $A_2$ being at least partially covered with at least one particulate solid UV-screening agent as defined in the preceding claims.

**7.** Composition according to Claim 6, in which the coating of the particles $A_1$ or $A_2$ also comprises at least one lipophilic or hydrophilic additional organic UV-screening agent and/or at least one pulverulent colorant.

**8.** Composition according to Claim 6 or 7, in which the spherical composite particles A comprise:

i) spherical particles $A_1$ having a mean size of greater than 0.1 μm and less than 1 μm, more preferentially less than 0.6 μm and even more preferentially less than 0.4 μm, the surface of said particles $A_1$ being at least partially covered with titanium dioxide particles and the core of the particles being constituted of styrene/methyl methacrylate copolymer;
ii) spherical particles $A_2$ having a mean size of greater than or equal to 2 μm, preferably greater than or equal to 3 μm, more preferentially greater than 4 μm and even better still greater than or equal to 5 μm, the surface of said particles $A_2$ being covered with at least titanium dioxide particles and the core of the particles being constituted of poly(methyl methacrylate).

**9.** Composition according to Claim 6 or 7, in which the spherical composite particles A comprise:

i) spherical particles $A_1$ having a mean size of greater than 0.1 μm and less than 1 μm more preferentially less than 0.6 μm and even more preferentially less than 0.4 μm, the surface of said particles $A_1$ being at least partially covered with particles of particulate organic UV-screening agent and the core of the particles being constituted of styrene/methyl methacrylate copolymer;
ii) spherical particles $A_2$ having a mean size of greater than or equal to 2 μm, preferably greater than or equal to 3 μm, more preferentially greater than 4 μm and even better still greater than or equal to 5 μm, the surface of said particles $A_2$ being covered with at least particles of particulate organic UV-screening agent and the core of the particles being constituted of poly(methyl methacrylate).

**10.** Composition according to any one of Claims 1 to 9, in which the particles B of inorganic UV-screening agent are chosen from titanium dioxide ($TiO_2$) in rutile and/or anatase and/or amorphous form.

**11.** Composition according to any one of Claims 1 to 10, in which the inorganic UV-screening agent is in hydrophobically-modified form coated with

a) at least a first surface-treatment agent chosen from a dimethicone and a linear or branched $C_{12}$-$C_{18}$ fatty acid and more particularly stearic acid,
b) and optionally a second surface-treatment agent chosen from cerium oxide, alumina, silica, aluminium-based compounds such as aluminium hydroxide, silicon-based compounds, or mixtures thereof.

**12.** Composition according to any one of Claims 1 to 11, in which the inorganic UV-screening agent of the particles B is in the form of an oily dispersion and more particularly in the form of a dispersion in an oil chosen from $C_{12}$-$C_{15}$

alkyl benzoates or triglycerides.

13. Composition according to any one of Claims 1 to 12, **characterized in that** it is in the form of an aqueous lotion or of an aqueous gel or in the form of a simple or complex emulsion and more particularly in the form of a water-in-oil emulsion.

## Patentansprüche

1. Zusammensetzung, die in einem kosmetisch unbedenklichen Medium mindestens Folgendes enthält:

   a) mindestens eine wässrige Phase und
   b) Kompositpartikel A in Kugelform mit einer mittleren Größe zwischen 0,1 und 30 pm, die mindestens eine partikuläre UV-Filtersubstanz und einen Kern, der aus mindestens einem anorganischen Material und/oder mindestens einem organischen Material aufgebaut ist, umfassen, und
   c) freie Partikel B einer anorganischen UV-Filtersubstanz mit einer mittleren Elementargröße von größer als 0,07 μm,

   wobei der Gehalt an Kompositpartikeln A in der erfindungsgemäßen Zusammensetzung 1 % bis 70 %, bezogen auf auf das Gesamtgewicht der kosmetischen Zusammensetzung, beträgt,
   der Gehalt an Partikeln B in der erfindungsgemäßen Zusammensetzung 0,1 % bis 40 %, bezogen auf auf das Gesamtgewicht der Zusammensetzung, beträgt,
   der Kern der kugelförmigen Kompositpartikel A ein Material oder eine Mischung von Materialien enthält, die aus :

   - $SiO_2$,
   - Poly(methylmethacrylat),
   - Copolymeren von Styrol und einem C1/C5-Alkyl-(Meth)acrylat-Derivat ausgewählt sind,

   die partikuläre UV-Filtersubstanz anorganisch ist und aus Titanoxiden ausgewählt ist,
   die Partikel B einer anorganischen UV-Filtersubstanz Metalloxide, die mit mindestens einem Oberflächenbehandlungsmittel behandelt wurden, in hydrophob modifizierter gecoateter Form, die aus:

   - einem mit Dimethicon behandelten Titandioxid;
   - einem Titandioxid, das mit einer Mischung aus Siliciumdioxid/Aluminiumoxid/Stearinsäure oder Aluminiumoxid/Stearinsäure behandelt ist;
   - einer öligen Dispersion von $TiO_2$-Partikeln, die mit einer Mischung aus Stearinsäure, Polyhydroxystearinsäure und Aluminiumoxid behandelt ist, ausgewählt sind.

2. Zusammensetzung nach Anspruch 1, bei der die Kompositpartikel A einen Kern enthalten, der mindestens ein organisches Material und/oder mindestens ein anorganisches Material umfasst, in dem Partikel einer partikulären UV-Filtersubstanz enthalten sind.

3. Zusammensetzung nach Anspruch 1, bei der die kugelförmigen Kompositpartikel A einen Kern enthalten, der aus einem organischen Material und/oder einem anorganischen Material aufgebaut ist, der mit mindestens einer Schicht einer partikulären UV-Filtersubstanz, die mittels eines Bindemittels mit der Matrix verbunden sein kann, bedeckt ist.

4. Zusammensetzung nach Anspruch 1, bei der die Kompositpartikel A eine partikuläre UV-Filtersubstanz enthalten, die mit mindestens einer Schicht eines organischen Materials und/oder eines anorganischen Materials bedeckt sein kann.

5. Zusammensetzung nach einem der Ansprüche 1 bis 4, bei der der Gehalt an Kompositpartikeln A in der erfindungsgemäßen Zusammensetzung 1,5 Gew.-% bis 50 Gew.-% und vorzugsweise 2 Gew.-% bis 40 Gew.-%, bezogen auf auf das Gesamtgewicht der kosmetischen Zusammensetzung, beträgt.

6. Zusammensetzung nach einem der Ansprüche 1 bis 5, bei der die kugelförmigen Kompositpartikel A aus Folgendem aufgebaut sein können:

   i) kugelförmige Partikel $A_1$ mit einer mittleren Größe von größer als 0,1 μm und weniger als 1 pm, weiter

bevorzugt weniger als 0,6 µm und noch weiter bevorzugt weniger als 0,4 pm, wobei die Oberfläche der Partikel $A_1$ mindestens zum Teil mit mindestens einer partikulären festen UV-Filtersubstanz gemäß einem der vorhergehenden Ansprüche bedeckt ist;
ii) und gegebenenfalls kugelförmige Partikel $A_2$ mit einer mittleren Größe von größer oder gleich 2 pm, vorzugsweise größer oder gleich 3 pm, weiter bevorzugt größer als 4 µm und noch besser größer oder gleich 5 pm, wobei die Oberfläche der Partikel $A_2$ mindestens zum Teil mit mindestens einer partikulären festen UV-Filtersubstanz gemäß einem der vorhergehenden Ansprüche bedeckt ist.

**7.** Zusammensetzung nach Anspruch 6, bei der der Überzug der Partikel $A_1$ oder $A_2$ auch mindestens eine zusätzliche lipophile oder hydrophile organische UV-Filtersubstanz und/oder mindestens ein pulverförmiges Farbmittel umfasst.

**8.** Zusammensetzung nach Anspruch 6 oder 7, bei der die kugelförmigen Kompositpartikel A Folgendes umfassen:

i) kugelförmige Partikel $A_1$ mit einer mittleren Größe von größer als 0,1 µm und weniger als 1 pm, weiter bevorzugt weniger als 0,6 µm und noch weiter bevorzugt weniger als 0,4 pm, wobei die Oberfläche der Partikel $A_1$ mindestens zum Teil mit Titandioxidpartikeln bedeckt ist und der Kern der Partikel aus einem Styrol-Metyhlmethacrylat-Copolymer aufgebaut ist;
ii) kugelförmige Partikel $A_2$ mit einer mittleren Größe von größer oder gleich 2 pm, vorzugsweise größer oder gleich 3 pm, weiter bevorzugt größer als 4 µm und noch besser größer als 5 µm, wobei die Oberfläche der Partikel $A_2$ wenigstens mit Titandioxidpartikeln bedeckt ist und der Kern der Partikel aus Poly(metyhlmethacrylat) aufgebaut ist;

**9.** Zusammensetzung nach Anspruch 6 oder 7, bei der die kugelförmigen Kompositpartikel A Folgendes umfassen:

i) kugelförmige Partikel $A_1$ mit einer mittleren Größe von größer als 0,1 µm und weniger als 1 pm, weiter bevorzugt weniger als 0,6 µm und noch weiter bevorzugt weniger als 0,4 pm, wobei die Oberfläche der Partikel $A_1$ mindestens zum Teil mit Partikeln einer organischen UV-Filtersubstanz bedeckt ist und der Kern der Partikel aus einem Styrol-Metyhlmethacrylat-Copolymer aufgebaut ist;
ii) kugelförmige Partikel $A_2$ mit einer mittleren Größe von größer oder gleich 2 pm, vorzugsweise größer oder gleich 3 pm, weiter bevorzugt größer als 4 µm und noch besser größer oder gleich 5 pm, wobei die Oberfläche der Partikel $A_2$ wenigstens mit Partikeln einer partikulären organischen UV-Filtersubstanz bedeckt ist und der Kern der Partikel aus Poly(metyhlmethacrylat) aufgebaut ist.

**10.** Zusammensetzung nach einem der Ansprüche 1 bis 9, bei der die Partikel B einer anorganischen UV-Filtersubstanz aus Titandioxid ($TiO_2$) in Rutil- und/oder Anatasform und/oder amorpher Form ausgewählt sind.

**11.** Zusammensetzung nach einem der Ansprüche 1 bis 10, bei der die anorganische UV-Filtersubstanz in hydrophob modifizierter Form vorliegt und mit Folgendem überzogen ist: a) wenigstens einem ersten Oberflächenbehandlungsmittel, das aus Dimethicon und einer linearen oder verzweigten $C_{12}$-$C_{18}$-Fettsäure und insbesondere Stearinsäure ausgewählt ist, b) und gegebenenfalls einem zweiten Oberflächenbehandlungsmittel, das aus Ceroxid, Aluminiumoxid, Siliciumdioxid, Verbindungen auf Aluminiumbasis wie Aluminiumhydroxid, Verbindungen auf Siliciumbasis oder Mischungen davon ausgewählt ist.

**12.** Zusammensetzung nach einem der Ansprüche 1 bis 11, bei der die anorganische UV-Filtersubstanz der Partikel B in Form einer öligen Dispersion und insbesondere in Form einer Dispersion in einem Öl, das aus $C_{12}$-$C_{15}$-Alkylbenzoaten oder Triglyceriden ausgewählt ist, vorliegt.

**13.** Zusammensetzung nach einem der Ansprüche 1 bis 12, **dadurch gekennzeichnet, dass** sie in Form einer wässrigen Lotion oder eines wässrigen Gels oder in Form einer einfachen oder komplexen Emulsion und insbesondere in Form einer Wasser-in-Öl-Emulsion vorliegt.

## Revendications

**1.** Composition contenant, dans un milieu acceptable sur le plan cosmétique, au moins :

a) au moins une phase aqueuse et
b) des particules composites A sous forme sphérique, possédant une taille moyenne comprise entre 0,1 et 30

μm comprenant au moins un agent anti-UV particulaire et un noyau constitué d'au moins un matériau inorganique et/ou d'au moins un matériau organique, et

c) des particules libres B d'agent anti-UV inorganique possédant une taille moyenne élémentaire supérieure à 0,07 pm,

la teneur en particules composites A dans la composition selon l'invention se situant dans la plage de 1 % à 70 % par rapport au poids total de la composition cosmétique,
la teneur en particules B dans la composition selon l'invention se situant dans la plage de 0,1 % à 40 % par rapport au poids total de la composition,
le noyau des particules composites sphériques A contenant un matériau ou un mélange de matériaux choisis parmi :

- $SiO_2$,
- un poly(méthacrylate de méthyle),
- des copolymères de styrène et d'un dérivé de (méth)acrylate de C1/C5 alkyle,

l'agent anti-UV particulaire étant inorganique et choisi parmi des oxydes de titane,
les particules B d'agent anti-UV inorganique étant des oxydes métalliques traités avec au moins un agent de traitement de surface, sous forme revêtue modifiée de manière hydrophobe, choisis parmi :

- du dioxyde de titane traité par un diméthicone ;
- du dioxyde de titane traité avec un mélange silice/alumine/acide stéarique ou alumine/acide stéarique ;
- une dispersion huileuse de particules de $TiO_2$ traitées avec un mélange d'acide stéarique, de poly(acide hydroxystéarique) et d'alumine.

**2.** Composition selon la revendication 1, dans laquelle les particules composites A contiennent un noyau comprenant au moins un matériau organique et/ou au moins un matériau inorganique, dans lequel sont comprises des particules d'agent anti-UV particulaire.

**3.** Composition selon la revendication 1, dans laquelle les particules composites sphériques A contiennent un noyau fait d'un matériau organique et/ou d'un matériau inorganique, recouvert avec au moins une couche d'agent anti-UV particulaire qui peut être reliée à la matrice au moyen d'un liant.

**4.** Composition selon la revendication 1, dans laquelle les particules composites A contiennent un agent anti-UV particulaire recouvert avec au moins une couche d'un matériau organique et/ou d'un matériau inorganique.

**5.** Composition selon l'une quelconque des revendications 1 à 4, dans laquelle la teneur en particules composites A dans la composition selon l'invention se situe dans la plage de 1,5 % à 50 % et préférablement de 2 % à 40 % en poids par rapport au poids total de la composition cosmétique.

**6.** Composition selon l'une quelconque des revendications 1 à 5, dans laquelle les particules composites sphériques A peuvent être constituées :

i) de particules sphériques $A_1$ possédant une taille moyenne supérieure à 0,1 μm et inférieure à 1 pm, plus préférentiellement inférieure à 0,6 μm et encore plus préférentiellement inférieure à 0,4 μm, la surface desdites particules $A_1$ étant au moins partiellement recouverte avec au moins un agent anti-UV solide particulaire tel que défini dans les revendications précédentes ;
ii) et éventuellement de particules sphériques $A_2$ possédant une taille moyenne supérieure ou égale à 2 pm, préférablement supérieure ou égale à 3 μm, plus préférentiellement supérieure à 4 μm et même encore mieux supérieure ou égale à 5 μm, la surface desdites particules $A_2$ étant au moins partiellement recouverte avec au moins un agent anti-UV solide particulaire tel que défini dans les revendications précédentes.

**7.** Composition selon la revendication 6, dans laquelle le revêtement des particules $A_1$ ou $A_2$ comprend également au moins un agent anti-UV organique supplémentaire lipophile ou hydrophile et/ou au moins un colorant pulvérulent.

**8.** Composition selon la revendication 6 ou 7, dans laquelle les particules composites sphériques A comprennent :

i) des particules sphériques $A_1$ possédant une taille moyenne supérieure à 0,1 μm et inférieure à 1 pm, plus préférentiellement inférieure à 0,6 μm et encore plus préférentiellement inférieure à 0,4 μm, la surface desdites

particules $A_1$ étant au moins partiellement recouverte avec des particules de dioxyde de titane et le noyau des particules étant constitué d'un copolymère de styrène/méthacrylate de méthyle ;
ii) des particules sphériques $A_2$ possédant une taille moyenne supérieure ou égale à 2 pm, préférablement supérieure ou égale à 3 μm, plus préférentiellement supérieure à 4 μm et même encore mieux supérieure ou égale à 5 μm, la surface desdites particules $A_2$ étant recouverte avec au moins des particules de dioxyde de titane et le noyau des particules étant constitué de poly(méthacrylate de méthyle).

**9.** Composition selon la revendication 6 ou 7, dans laquelle les particules composites sphériques A comprennent :

i) des particules sphériques $A_1$ possédant une taille moyenne supérieure à 0,1 μm et inférieure à 1 pm, plus préférentiellement inférieure à 0,6 μm et encore plus préférentiellement inférieure à 0,4 μm, la surface desdites particules $A_1$ étant au moins partiellement recouverte avec des particules d'agent anti-UV organique particulaire et le noyau des particules étant constitué d'un copolymère de styrène/méthacrylate de méthyle ;
ii) des particules sphériques $A_2$ possédant une taille moyenne supérieure ou égale à 2 pm, préférablement supérieure ou égale à 3 pm, plus préférentiellement supérieure à 4 μm et même encore mieux supérieure ou égale à 5 pm, la surface desdites particules $A_2$ étant recouverte avec au moins des particules d'agent anti-UV organique particulaire et le noyau des particules étant constitué de poly(méthacrylate de méthyle).

**10.** Composition selon l'une quelconque des revendications 1 à 9, dans laquelle les particules B d'agent anti-UV inorganique sont choisies parmi du dioxyde de titane ($TiO_2$) sous forme de rutile et/ou d'anatase et/ou amorphe.

**11.** Composition selon l'une quelconque des revendications 1 à 10, dans laquelle l'agent anti-UV inorganique est sous une forme modifiée de manière hydrophobe revêtue avec :

a) au moins un premier agent de traitement de surface choisi parmi un diméthicone et un acide gras linéaire ou ramifié en $C_{12-18}$ et plus particulièrement l'acide stéarique,
b) et éventuellement un deuxième agent de traitement de surface choisi parmi l'oxyde de cérium, l'alumine, la silice, des composés à base d'aluminium tels que l'hydroxyde d'aluminium, des composés à base de silicium, et des mélanges correspondants.

**12.** Composition selon l'une quelconque des revendications 1 à 11, dans laquelle l'agent anti-UV inorganique des particules B est sous la forme d'une dispersion huileuse et plus particulièrement sous la forme d'une dispersion dans une huile choisie parmi des benzoates de $C_{12-15}$-alkyle ou des triglycérides.

**13.** Composition selon l'une quelconque des revendications 1 à 12, **caractérisée en ce qu'**elle est sous la forme d'une lotion aqueuse ou d'un gel aqueux ou sous la forme d'une émulsion simple ou complexe et plus particulièrement sous la forme d'une émulsion eau-dans-huile.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2882371
- WO 2006083326 A
- WO 9837964 A
- WO 2006061835 A
- EP 1388550 A
- WO 9822539 A
- WO 9522959 A
- WO 9703643 A
- GB 2286774 A
- EP 743309 A
- WO 9822447 A
- GB 2319523 A
- EP 0790243 A
- WO 9825922 A
- FR 2861075
- US 5687521 A
- US 5373037 A
- US 5362881 A
- US 5237071 A
- US 5166355 A
- GB 2303549 A
- DE 19726184
- EP 893119 A
- US 5888481 A
- DE 676103
- CH 350763
- US 5501850 A
- US 5961960 A
- EP 0669323 A
- US 5518713 A
- US 2463264 A
- EP 0921126 A
- EP 0712855 A
- JP 04134042 B
- JP 04134043 B
- EP 0576974 A
- FR 2395023
- JP 01158090 A
- EP 0390683 A
- JP 04134041 B
- FR 2506156
- EP 0693471 A
- FR 2528420
- FR 2529887
- EP 0694521 A
- FR 2638354
- EP 0714880 A
- JP 04290882 B
- FR 2639347 A
- JP 87166517 B
- WO 9310753 A
- WO 9311095 A
- WO 9505150 A
- EP 1353642 A
- US 5624663 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 04006878 A
- WO 05058269 A
- WO 06032741 A
- WO 2007068371 A
- WO 2008155059 A
- WO 9206778 A
- EP 1069142 A
- FR 2315991
- FR 2416008
- US 4077441 A
- US 4850517 A


### Non-patent literature cited in the description


- *Cosmetics & Toiletries,* February 1990, vol. 105, 53-64
- *J. Am. Chem. Soc.,* 1957, vol. 79, 5706-5708
- *J. Am. Chem. Soc.,* 1960, vol. 82, 609-611
- *J. Am.Chem. Soc.,* 1960, vol. 82, 609
- *J. Chim. Phys.,* 1967, vol. 64, 1602
- **E. MARIANI et al.** *16th IFSCC Congress,* 1990
- International Cosmetic Ingredient Dictionary and Handbook. The Cosmetic, Toiletries and Fragrance Association, 1997, 371-386, 524-528

- **BANGHAM ; STANDISH ; WATKINS.** *J. Mol. Biol.,* 1965, vol. 13, 238
- *J. Soc. Cosm. Chem.,* 1954, vol. 5, 249-256
- HLB Index. McCutcheon's Emulsifiers & Detergents. MC Publishing Company, 1998
- **B. L. DIFFEY.** *J. Soc. Cosmet. Chem.,* 1989, vol. 40, 127-133